# EUROPEAN PATENT APPLICATION

(11) **EP 3 025 733 A1**
(43) Date of publication of application: **01.06.2016**
(21) Application number: 14829240.2
(22) Date of filing: 08.02.2014
(51) Int. Cl.: A61K 47/48, A61K 31/7048, A61P 31/10

(54) **PHARMACEUTICAL, WATER-SOLUBLE AND ANTIFUNGAL MACROMOLECULAR COMPOUND**

(30) Priority: 25.07.2013 CN 201310315504
(71) Applicant: Beijing Labworld Bio-medicine Technology Corp. Ltd., Beijing 101318 (CN)
(72) Inventor: WEN, Guanghui, Beijing 101318 (CN); WAN, Liuyi, Beijing 101318 (CN); YU, Dongfang, Beijing 101318 (CN); YANG, Jingwen, Beijing 101318 (CN); WANG, Haojun, Beijing 101318 (CN)
(74) Representative: Zardi, Marco
(86) International application number: PCT/CN2014/000146
(87) International publication number: WO 2015/010439

(57) **Abstract**

The present invention relates to a medicinal water-soluble antifungal macromolecular compound used for treating infectious diseases, especially, relates to a poly-conjugate which includes an active drug substance and a macro-molecular polymer which are combined with each other through chemical bonds. The active drug substance is a polyene macrolide compound such as amphotericin B. Also provided are a method of preparing the poly-conjugate, a method for increasing solubility of the active drug substance and a method for treating fungal infection, which is any disease related to fungus, such as sepsis, endocarditis, meningitis (caused by cryptococcus and other fungus), abdominal infection (including those related to dialysis), pulmonary infection, urinary tract infection and the like. The poly-conjugate has excellent properties in biological performance and/or physical performance and/or chemical performance.

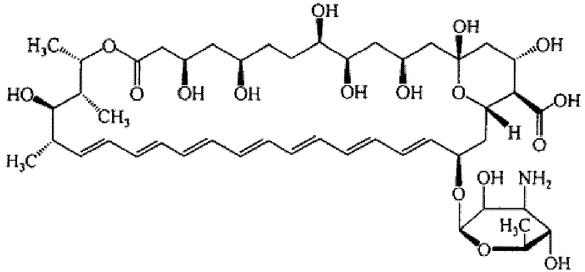

## Description

### Technical Field

The present invention relates to a medical drug for treating fungal infection, specifically a polyconjugate composed of a polyene macrolide antibiotic for treating infectious diseases, such as amphotericin B, and polymeric macropeptides or polysaccharides, more specifically a polyene macrolide polyconjugate, such as an amphotericin B polyconjugate, which is derived from combination reaction between polymeric macropeptides, polysaccharides or glycopeptides and polyene macrolide compounds, such as amphotericin B.

### Background

Polyene compounds (referred to herein also as polyene antibiotics, polyene macrolide antibiotics, polyenes, polyene anti-fungal drugs, etc.) is a class of macrolide compounds with conjugated polyene and hydroxyl. Typical examples of polyene compounds are amphotericin B, nystatin, natamycin, hachimycin, mepartricin, cannitracin, filipin, aureofungin, etruscomycin, hamycin, perimycin, etc. Polyene antibiotics were the first anti-fungal drugs used clinically. Their anti-fungal mechanism of action involves binding to the sterol on the cell membrane of susceptible fungus, damaging cell membrane permeability, causing leakage of important intracellular substances such as potassium ion, nucleotides and amino acids etc., interrupting cell metabolism and thereby inhibiting cell growth.

Amphotericin B (referred to herein also as AB as an abbreviation), which has been included in Chinese Pharmacopoeia 2010, has the following structural formula, molecular formula, and molecular weight:

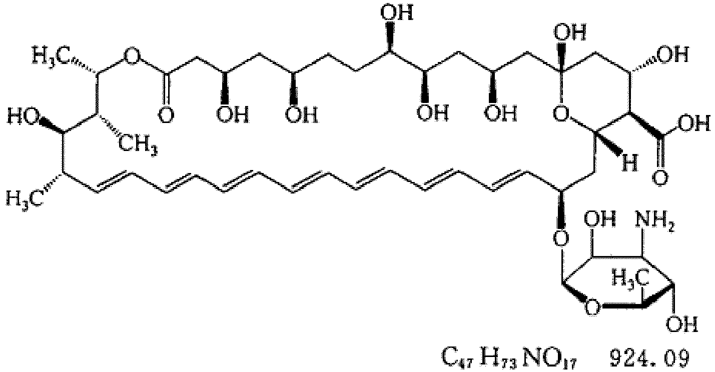

Amphotericin B is clinically used to treat fungal infection. It inhibits the growth of fungus by influencing the permeability of fungal cell membrane, and is most often used for treating severe organ or systemic deep fungal infection. Amphotericin B is a polyene compound isolated from the culture medium of Streptomyces nodosus. Chemically, it belongs to polyene macrolide substance, within its 38-membered lactonic ring, there are 7 conjugated double bonds all in trans configuration, the lactonic ring is further linked to aminosugar D-trehalosamine by O-glycosidic bond. Amphotericin B is soluble in dimethyl sulfoxide, slightly soluble in dimethylformamide, very slightly soluble in methanol, and insoluble in water, absolute ethanol, chloroform or ether. It can form a salt in neutral or acidic medium, with increased solubility but decreased anti-fungal activity. Amphotericin B is amphiprotic, with both lipophilic and hydrophilic region, which enables it to form a complex with sterols in the fungal cell membrane, causing damage to cell membrane permeability. Since none sterol exists in the cell membrane of bacteria, the antibiotic action of amphotericin B is selectively directed against fungus.

Amphotericin B is the first-choice drug for treating systemic fungal infection in human, as it displays a broad anti-fungal spectrum against practically all pathogenic fungus. Cases of invasive fungal infection surged in the recent years particularly in patients with compromised immune system, such as HIV or cancer patients.

On the other hand, treatment with amphotericin B is accompanied with side effects, sometimes severe. For treating systemic and organ fungal infection, the common daily dose for amphotericin B is 0.5-0.7 mg/kg body weight (i.v.). However, the dose should be adjusted or modified individually because different patient shows different tolerance towards the drug. Furthermore, patients with compromised immune system often require higher doses, such as 1 mg/kg body weight daily, and if well tolerated the required doses may increase up to 1.5 mg/kg in severe cases. Depending on the tolerability and severity of the infection, treatment with parenteral amphotericin B may last for several weeks to several months.

Typical infusion reactions are common during the parenteral therapy, such as fever, vomit and chills, which only need symptomatic treatment rather than interruption of the therapy. However, the liver and particularly kidney dysfunction are more severe infusion reactions and occurs frequently. For example, at the beginning of treatment, glomerular filtration rate always reduces by about 40%. Most patients will maintain such a low glomerular filtration rate during the entire treatment period. Therefore, the level of serum creatinine and blood urea increases. Occasionally, irreversible damage is observable post treatment/after the treatment is finished. Anemia often appears 2 to 3 weeks after treatment initiation, which reduces the hematocrit to 25-30%. However, the change in blood cell count is completely reversible after completion of therapy.

Because of its toxicity and the side effects, amphotericin B should only be used in case of a life-threatening fungal infection. Nevertheless, amphotericin B is usually the only drug effective against fungal diseases in immunocompromised patients (for example, AIDS or post-organ transplantation).

Amphotericin B has a significant hydrophobic property overall, even though it has hydrophilic regions in the molecule structure, thus under physiological pH, it is almost insoluble in water and only slightly soluble in organic solvents. Therefore, the current commercially available products are in drug forms having complex structures which are hampered by additional disadvantages. The water solubility could be enhanced by using suitable solubilizers, such as sodium deoxycholate. For example, the original infusion preparation manufactured by BRISTOL-MYERS SQUIBB (obtainable by trade name "Amphotericin B" in German) is in the form of lyophilized powder, which must be redissolved in water to form a a micellar dispersion of amphotericin B and sodium deoxycholate. The stock solution could be diluted to the desired final concentration by an electrolyte free liquid, such as 5% glucose solution, in order to obtain the ready-to-use infusion solution.

The above preparation is further characterized by a very limited therapeutic index, i.e., the range between effective and toxic dosage is very narrow. Furthermore, in certain clinical cases, such a preparation of amphotericin B has poor effect despite the broad action spectrum of amphotericin B itself, the lack or the insufficiency of the proper anti-fungal effect of amphotericin B in the fungal infected site is due to the inability for the preparation to deliver the active substance to the infected site and/or the lack of sufficient concentration of the active substance at the fungal infection site.

To overcome these disadvantages of the original preparation, a series of lipid based preparations of amphotericin B has been developed, for example, a lipid complex of lipids and amphotericin B, a colloidal dispersion of cholesterol sulfate and amphotericin B, and amphotericin B contained in liposome. These drug forms all have improved therapeutic index and tolerance, especially with lower nephrotoxicity compared with the conventional sodium deoxycholate preparation of amphotericin B, therefore, these drug forms can be administered in higher dosage without completely avoiding the side effects.

Another major disadvantage of the amphotericin B preparations is the high product cost, which leads to the high price thereof. Further, these complicated drug forms are inconvenient since they must be re-dissolved in order to obtain the ready-to-use form. Despite some improvement in their therapeutic index, the lipid based preparations of amphotericin B have not been widely accepted by the marketdue to the above disadvantages and other problems.

The clinical efficacy of amphotericin B has long been established. Its clinical use is however limited due to the following: 1. low water solubility, lack of suitable formulation for intravenous administration; 2. high toxic and side effect, limited dosage range; 3. because of the limited dosage range, dose has to be increased gradually, which would compromise the therapeutic effect while increase the chance of developing clinical resistance.

The amphotericin B preparations which are currently available for clinical use mainly include:

Amphotericin B for injection, the first amphotericin B preparation used in clinic, which uses sodium deoxycholate as a solubilizer to form colloidal dispersion after re-dissolution, such as the one manufactured by NORTH CHINA PHARMACEUTICAL COMPANY. LTD (National Medicine Permission Number H13020283). Indications include: deep and progressively deteriorating fungal infection caused by susceptible fungus and, such as septicemia, endocarditis, meningitis (Cryptococcus sp. and other fungus), abdominal infection (including those undergoing dialysis), pulmonary infection, urinary tract infection and endophthalmitis, etc;

Amphotericin B liposome for injection, which is a liposomal preparation made of cholesteryl sodium sulfate, tromethamine, disodium EDTA, lactose monohydrate, and hydrochloric acid, such as the imported product from Three Rivers Pharmaceuticals.LLC (trade name: Amphotec, Permission Number H20090964). Indications of the liposomal preparation include: patients having deep fungal infection, patients whose renal impairment or drug toxicity precludes the use of effective dose of the conventional amphotericin B, patients failing conventional amphotericin B therapy;

Amphotericin B lipid complex injection, for example, product ABELCET® (trade name) manufactured by Sigma-tau pharmaceutic company, is a sterile, pyrogenic-free suspension for intravenous infusion. It consists of amphotericin B complexed with two lipid components in a 1:1 drug-to-lipid molar ratio. The two lipid components, L-α-dimyristoylphosphatidylcholine (DMPC) and L-α-dimyristoyl phosphatidyl glycerol (DMPG), are present in a 7:3 molar ratio. The infusion is indicated for the treatment of invasive fungal infections in patients who are refractory to or intolerant of conventional amphotericin B therapy.

Amphotericin B liposome for injection, AmBisome®, is a sterile, non-pyrogenic lyophilized product for intravenous infusion. Each vial contains 50 mg amphotericin B, intercalated into a liposomal membrane consisting of 213 mg hydrogenated soy phosphatidylcholine, 52 mg cholesterol, 84 mg distearoyl phosphatidyl glycerol, 0.64 mg αtocopherol, together with 900 mg sucrose and 27 mg disodium succinate hexahydrate. AmBisome® is indicated for the empirical therapy for presumed fungal infection in febrile, neutropenic patients, treatment of Cryptococcal Meningitis in HIV infected patients, treatment of patients with Aspergillus species, Candida species and/or Cryptococcus species infections refractory to amphotericin B deoxycholate, or in patients whose renal impairment or unacceptable toxicity precludes the use of amphotericin B deoxycholate. Therefore, the method (formulation) of using liposome as carrier to overcome some disadvantages of amphotericin B has been widely accepted clinically.

However, in the product descriptions of the above amphotericin B lipid complex injection ABELCET® and AmBisome®, the following precautions are explicitly specified (in bold): liposomal encapsulation or incorporation in a lipid complex can substantially affect a drug's functional properties relative to those of the unencapsulated or nonlipid-associated drug. In addition, different liposomal or lipid-complex products with a common active ingredient may vary from one another in the chemical composition and physical form of the lipid component. Such differences may have an effect on properties of these drug products (see http://www.rxlist.com/abelcet-drug.htm). Therefore, it is well-known in the field that for the same active drug substance such as amphotericin B, different drug carrier can lead to totally different, unpredictable physicochemical or even biological properties to the drug.

Considering the numerous deficiencies associated with the clinical use of amphotericin B, a person skilled in the art still expects to provide a method of treating infectious diseases, such as those caused by fungal infection. For example, it is expected to provide a method for treating such diseases by using amphotericin B as active substance, and it is expected that the method, as well as the therapeutics provided by the method, have advantages in one or more aspects.

### SUMMARY OF THE INTENTION

An object of the present invention is to provide a method of treating infectious diseases, such as those caused by fungal infection. For example, it is expected to provide a method for treating such diseases by using amphotericin B as active substance, and it is expected that the method, as well as the therapeutic agents provided by the method, have advantages in one or more aspects. In the present invention, it has been surprisingly found that the amphotericin B polyconjugates displays at least one unexpected property, such as physicochemical property and/or biological property, wherein the polyconjugates are obtained by combining polymeric macro-molecule with amphotericin B. It has also been surprisingly found that other polyene macrolide antibiotics when so combined with the polymeric macro-molecule show similar unexpectedly properties as those conferred to amphotericin B. The present invention is accomplished based on these findings.

Therefore, the first aspect of the present invention provides a pharmaceutically acceptable polyconjugate comprising an active drug substance and a macro-molecular polymer, wherein the active drug substance is conjugated with the macro-molecular polymer by chemical bonds.

According to an embodiment of the first aspect, the present invention provides the polyconjugate, wherein the active drug substance is a polyene macrolide antibiotic.

According to an embodiment of the first aspect, the present invention provides the polyconjugate, wherein the active drug substance is selected from the following polyene macrolide antibiotics: amphotericin B, nystatin, natamycin, trichomycin, mepartricin, cannitracin (CAS No.84930-92-7, for example, the product obtained from ZHEJIANG HAIZHENG PHARM CO LTD, National Medicine Permission Number H33021810, filipin III (CAS No. 480-49-9), aureofungin, etruscomycin, hamycin (CAS No. 1403-71-0), perimycin A (CAS No. 62327-61-1), etc. In one embodiment, the active drug substance is selected from the following polyene macrolide antibiotics: amphotericin B, nystatin, natamycin, trichomycin.

According to an embodiment of the first aspect, the present invention provides the polyconjugate, wherein the macro-molecular polymer is selected from poly-amino acid formed by polymerizing amino acids, and poly-aminohexose formed by polymerizing aminohexoses.

According to an embodiment of the first aspect, the present invention provides the polyconjugate, wherein the macro-molecular polymer is poly-amino acid formed by polymerizing amino acids. The amino acid is selected from carboxylic acid-type amino acid, amino-type amino acid, or the combination thereof, thus forming carboxylic acid-type poly-amino acid or amino-type poly-amino acid.

According to an embodiment of the first aspect, the present invention provides the polyconjugate, wherein the carboxylic acid-type amino acid is selected from aspartic acid (Asp), glutamate (Glu), or the combinations thereof at any proportion.

According to an embodiment of the first aspect, the present invention provides the polyconjugate, wherein the amino-type amino acid is selected from ornithine (Orn), lysine (Lys), arginine (Arg), hydroxylysine (Hyl), or the combinations thereof at any proportion.

According to an embodiment of the first aspect, the present invention provides the polyconjugate, wherein the amino acids in the poly-amino acid comprise D-amino acid, L-amino acid, and DL-amino acid.

According to an embodiment of the first aspect, the present invention provides the polyconjugate, wherein the aminohexose is selected from glucosamine, galactosamine, acetyl glucosamine, acetyl galactosamine, or the combinations thereof, thus forming poly-glucosamine, poly-galactosamine, poly-acetylglucosamine, poly-acetylgalactosamine and the like.

According to an embodiment of the first aspect, the present invention provides the polyconjugate, wherein the average molecular weight of the poly-amino acid is 5000-100000 daltons, for example, 10000-80000 daltons, 10000-50000 daltons, 10000-40000 daltons.

According to an embodiment of the first aspect, the present invention provides the polyconjugate, wherein the average molecular weight of the poly-aminohexose is 10000-200000 daltons, for example, 20000-180000 daltons, 20000-150000 daltons, 30000-120000 daltons, 40000-100000 daltons.

According to an embodiment of the first aspect, the present invention provides the polyconjugate, wherein the weight of the active drug substance accounts for 5-60% of the total weight of the polyconjugate, i.e., the polyconjugate contains 5-60 weight% active drug substance and the rest is the macro-molecular polymer. For example, the weight of the active drug substance accounts for 10-50%, 10-40%, 10-35% of the total weight of the polyconjugate.

According to an embodiment of the first aspect, the present invention provides the polyconjugate, wherein the polyconjugate could use water for injection, sodium chloride injection, or glucose injection as the injection solvent at the time of administration.

Furthermore, the second aspect of the present invention provides a method of preparing a polyconjugate according to an embodiment of the first aspect of the present invention, including the following steps:
i) providing a macro-molecular polymer, dissolving it into a solvent to form a solution;
ii) adding and dissolving the active drug substance into the solution obtained in step (i);
iii) adding catalyst and condensing agent into the solution obtained in step (ii), condensing the macro-molecular polymer with the active drug substance to form the polyconjugate thereof, wherein the macro-molecular polymer and the active drug substance are combined with each other by chemical bonds;
(iv) separating and extracting the obtained polyconjugate.

According to an embodiment of the second aspect, the present invention provides the method, wherein the solvent used in step (i) is an organic solvent, such as but not limited to dimethylformamide, dimethyl sulfoxide and the like.

According to an embodiment of the second aspect, the present invention provides the method, wherein the catalyst used in step (iii) is 4-dimethylaminopyridine (abbreviated as DMAP). The amount of the catalyst usually can be 0.2-4 times of the molar amount of the active drug substance, such as 0.5-2, 0.8-1.5, or 1.0-1.2 times.

According to an embodiment of the second aspect, the present invention provides the method, wherein the condensing agent used in step (iii) is dicyclohexylcarbodiimide (abbreviated as DCC). The amount of the condensing agent usually can be 0.5-2 times of the molar amount of the active drug substance, such as 0.8-1.5 or 1.0-1.2 times.

According to an embodiment of the second aspect, the present invention provides the method, wherein the separating and extracting of step (iv) is carried out by dialysis-based method, which can easily remove the unbound, free active drug substance. This is well known to a person skilled in the art.

In the method of the present invention, the free carboxyl group in the carboxylic acid-type poly-amino acid (such as poly-aspartic acid) can condense with the hydroxyl group in the active drug molecule under the reaction condition using condensing agent DCC and catalyst DMAP, in order to form the polyconjugate composed of polyene antibiotic and poly-amino acid that are combined with each other through chemical bond. In addition, the above reaction condition using condensing agent DCC and catalyst DMAP can also enable the free amino group in the amino-type poly-amino acid, such as poly-ornithine, to condense with the carboxyl group in the active drug molecule (especially those containing the carboxyl group), in order to form the polyconjugate composed of polyene antibiotic and such poly-amino acid that are combined with each other through chemical bond. In addition, the above reaction condition using condensing agent DCC and catalyst DMAP can also enable the carboxyl group in the poly-aminohexose, such as poly-glucosamine, to condense with the carboxyl group in the active drug molecule (especially those containing the carboxyl group), in order to form the polyconjugate composed of polyene antibiotic and such poly-aminohexose that are combined with each other through chemical bond. Other method known by the person skilled in the art that can combine the active drug substance with the macro-molecular polymer through chemical bonds can also be used in the present invention.

It has surprisingly been found that, by means of the method disclosed herein, the amphotericin B polyconjugate of the present invention has at least been conferred with an unexpectedly higher water solubility compared to that of the corresponding active drug substance. For example, by means of the method, the obtained amphotericin B polyconjugate (as calculated in terms of equivalent amphotericin B) has an unexpectedly higher solubility compared to that of the prototype amphotericin B.

The third aspect of the present invention further provides a method of increasing the solubility of an active drug substance, comprising the step of combining an active drug substance with a macro-molecular polymer by chemical bonds to form a polyconjugate.

According to an embodiment of the third aspect, the present invention provides the method, wherein the active drug substance is a polyene macrolide antibiotic.

According to an embodiment of the third aspect, the present invention provides the method, wherein the active drug substance is selected from the following polyene macrolide antibiotics: amphotericin B, nystatin, natamycin, trichomycin, mepartricin, cannitracin (CAS No.84930-92-7, for example, the product obtained from ZHEJIANG HAIZHENG PHARM CO LTD, National Medicine Permission Number H33021810, filipin III (CAS No. 480-49-9), aureofungin, etruscomycin, hamycin (CAS No. 1403-71-0), perimycin A (CAS No. 62327-61-1), etc. In one embodiment, the active drug substance is selected from the following polyene macrolide antibiotics: amphotericin B, nystatin, natamycin, trichomycin.

According to an embodiment of the third aspect, the present invention provides the method, wherein the macro-molecular polymer is selected from poly-amino acid formed by polymerizing amino acids, and poly-aminohexose formed by polymerizing aminohexoses.

According to an embodiment of the third aspect, the present invention provides the method, wherein the macro-molecular polymer is poly-amino acid formed by polymerizing amino acids. The amino acid is selected from carboxylic acid-type amino acid, amino-type amino acid, or the combination thereof, thus forming carboxylic acid-type poly-amino acid or amino-type poly-amino acid.

According to an embodiment of the third aspect, the present invention provides the method, wherein the carboxylic acid-type amino acid is selected from aspartic acid (Asp), glutamate (Glu), or the combinations thereof at any proportion.

According to an embodiment of the third aspect, the present invention provides the method, wherein the amino-type amino acid is selected from ornithine (Orn), lysine (Lys), arginine (Arg), hydroxylysine (Hyl), or the combinations thereof at any proportion.

According to an embodiment of the third aspect, the present invention provides the method, wherein the amino acids in the poly-amino acid comprise D-amino acid, L-amino acid, and DL-amino acid.

According to an embodiment of the third aspect, the present invention provides the method, wherein the aminohexose is selected from glucosamine, galactosamine, acetyl glucosamine, acetyl galactosamine, or the combinations thereof, thus forming poly-glucosamine, poly-galactosamine, poly-acetylglucosamine, poly-acetylgalactosamine and the like.

According to an embodiment of the third aspect, the present invention provides the method, wherein the average molecular weight of the poly-amino acid is 5000-100000 daltons, for example, 10000-80000 daltons, 10000-50000 daltons, 10000-40000 daltons.

According to an embodiment of the third aspect, the present invention provides the method, wherein the average molecular weight of the poly-aminohexose is 10000-200000 daltons, for example, 20000-180000 daltons, 20000-150000 daltons, 30000-120000 daltons, 40000-100000 daltons.

According to an embodiment of the third aspect, the present invention provides the method, wherein the weight of the active drug substance accounts for 5-60% of the total weight of the polyconjugate, i.e., the polyconjugate contains 5-60 weight% active drug substance and the rest is the macro-molecular polymer. For example, the weight of the active drug substance accounts for 10-50%, 10-40%, 10-35% of the total weight of the polyconjugate.

According to any embodiment of the third aspect, the present invention provides the method, wherein the polyconjugate could use water for injection, sodium chloride injection, or glucose injection as the injection solvent at the time of administration.

According to any embodiment of the third aspect, the present invention provides the method, wherein the step of combining an active drug substance with a macro-molecular polymer by chemical bonds to form a polyconjugate may be carried out in accordance with the method according to any embodiment of the second aspect of the present invention.

Base on the unexpected effect of the method according to the third aspect of the present invention, the inventors have also found that the polyconjugate obtained by the method of improving solubility of an active drug substance has additional unexpected superior properties that are completely unpredictable by one or more currently known techniques in the art.

Furthermore, the fourth aspect of the present invention provides a pharmaceutical composition, which comprises a polyconjugate of any embodiment of the first aspect of the present invention, and optionally pharmaceutically acceptable auxiliary material.

According to an embodiment of the fourth aspect, the present invention provides the pharmaceutical composition in any form of drug preparation in the pharmaceutical industry, such as but not limited to oral forms such as tablet, capsule, granule, oral solution, oral suspension, etc., injection forms such as small volume injection, large volume injection and sterile powder (e.g., freeze dried powder for injection, etc.), inhalant such as nasal inhalant (e.g., inhalable powder or inhalable solution, etc.).

The choice of the auxiliary material depends on the specific form of the pharmaceutical composition. For example, when the pharmaceutical composition is in the form of tablet, capsule, granule and the like, it can optionally include diluent (e.g., starch, lactose, microcrystalline cellulose, etc), disintegrant (e.g., sodium carboxymethyl cellulose, sodium carboxymethyl starch, low substituent hydroxypropyl cellulose, etc), adhesive (e.g., hydroxypropyl methylcellulose, polyvinylpyrrolidone, polyethylene glycol, etc), lubricant or glidant (e.g., magnesium stearate, talc powder, stearic acid, silica gel, etc). Optionally, the pharmaceutical composition can further include flavoring agent, coloring agent, coating agent and the like.

For example, when the pharmaceutical composition is in the form of oral solution or oral suspension and the like, it can include solvent, such as water, ethanol, glycerin, propanediol, polyethylene glycol (molecular weight of 200-600), etc. Optionally, the pharmaceutical composition can further include flavoring agent, coloring agent and the like.

For example, when the pharmaceutical composition is in the form of small volume injection, large volume injection and the like, it can include solvent, such as water, ethanol, glycerin, propanediol, polyethylene glycol (molecular weight of 200-600), etc. Optionally, the pharmaceutical composition can further include permeation pressure regulator, such as sodium chloride, glucose, etc.

For example, when the pharmaceutical composition is in the form of sterile powder, it can include excipient, such as mannitol, glucose, dextran, sodium chloride, sucrose and the like.

The preparation of these medicinal forms for the pharmaceutical composition of the present invention can be achieved by a person skilled in the art using well-known formula and preparation method.. For example, the tablet can be prepared through wet granulation tableting process, dry granulation tableting process, direct powder tableting process and the like. For example, the freeze dried powder injection can be prepared by adding proper amount of mannitol in accordance with the requirements, dissolving into water for injection, and freeze drying.

According to any embodiment of the fourth aspect, the present invention provides the pharmaceutical composition, which is the pharmaceutical composition for injection, and uses water for injection, 0.9% sodium chloride injection, or 5% glucose injection as the injection solvent at the time of administration.

Furthermore, the fifth aspect of the present invention provides a method of treating fungal infection related diseases, which comprises the step of delivering to a primate in need thereof an effective amount of the polyconjugate according to any embodiment of the first aspect of the present invention or the pharmaceutical composition according to any embodiment of the fourth aspect of the present invention.

According to an embodiment of the fifth aspect, the present invention provides the method, wherein the fungal infection related diseases can be any disease related to fungus, such as any known disease suitable for being treated by amphotericin B preparation, such as confirmed deep and/or deteriorating fungal infection by susceptible fungus, for example, sepsis, endocarditis, meningitis (caused by Cryptococcus and other fungus), abdominal infection (including those related to dialysis), pulmonary infection, urinary tract infection and the like.

Any embodiment of any aspect of the present invention can be combined with other embodiments, provided that they do not contradict each other. In addition, any technical feature in any embodiment of any aspect of the present invention can adapt to the technical feature in other embodiments, provided that they do not contradict each other.

Any technical feature of any aspect of the present invention or any embodiment of such aspect may apply to any other aspect or any embodiment of such other aspect, provided that they do not contradict each other. Of course, when mutually applicable, if necessary, the corresponding technical feature may be suitably modified. Each aspect and feature of the present invention is further described as follow.

The disclosures of each and every reference cited herein are incorporated herein in their entirety by reference, and if the meanings expressed in these references are different from those defined in the present invention, the expressions in the present invention shall prevail. In addition, all terms and phrases used herein have the same meaning as commonly understood by the person skilled in the art. Nevertheless, it is desired in the present invention to further illustrate and explain these terms and phrases in more detail. If the mentioned terms and phrases have meanings different from their common meanings, the meanings expressed in the present invention shall prevail.

The term "polyconjugate" (also referred to as "conjugate", "polymer", etc.) used herein refers to a chemical which comprises two moieties, an active drug substance and a macro-molecular polymer, wherein the active drug moiety is conjugated with the macro-molecular polymer moiety by chemical bonds, such as ester bond or amide bond. Therefore in nature, the polyconjugate disclosed in the present invention is a compound, i.e., it can be referred to as "compound". The term "poly" has the same meaning as commonly understood by a person skilled in the art, for example, it can be understood as multimer, polymer, high-molecular substance, high-molecular compound, polymeric macro-molecule and the like, such as the disclosed poly-amino acid (i.e., polypeptide) and poly-aminohexose (polysaccharide) and the like.

The active drug substance is a polyene macrolide antibiotic, also referred to as polyene macrolide substances, polyenes and the like. For example, amphotericin B, nystatin, natamycin, trichomycin, mepartricin, cannitracin, filipin, aureofungin, etruscomycin, hamycin (CAS No. 1403-71-0), perimycin, etc.

It is well known that amphotericin B has been included in many countries' drug standards, and is readily available in the market. Amphotericin B has the following structural formula, molecular formula, and molecular weight:

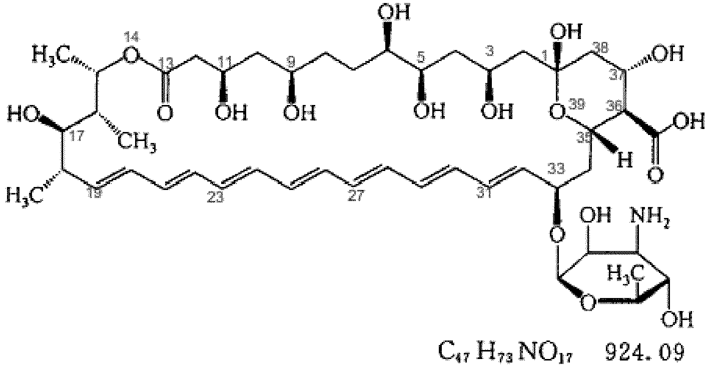

For example, in Chinese Pharmacopoeia 2010, amphotericin B is designated as [(1R-1R*,3S*,SR*,6R*,9R*,11R*,155*,16R*,17R*,185*,19E,21E,23E,25E, 27E,29E,31 E,33R*,355*,36R*,375*)]-33- [(3-amino-3,6-dideoxy-β-D-mamopyranosyl) oxy]-1,3,5,6,9,11,17,37-octahydroxy- 15,16,18-trimethyl- 13-oxo- 14,39-dioxabicyclo- [33.3.1] nonatriaconta- 19,21, 23,25,27,29,31-heptaene-36-carboxylic acid. Amphotericin B typically includes multiple hydroxyl groups and carboxyl groups; the hydroxyl group at 37^{th} position is particularly advantageous to the condensation reaction. The hydroxyl groups and the carboxyl groups both enable amphotericin B to bond with the disclosed macro-molecular polymer which has carboxyl group, amino group or hydroxyl group and form the polyconjugate. Of course, it is well-known to a person skilled in the art that other hydroxyl groups at positions other than position 37^{th}, also have the possibility of bonding with the carboxyl groups in the macro-molecule to form the polyconjugate of the present invention Such possibilities are included rather than being excluded herein.

In addition, nystatin (CAS No. 1400-61-9) is known to be included in British Pharmacopoeia, 2010 and has the following structural formula:

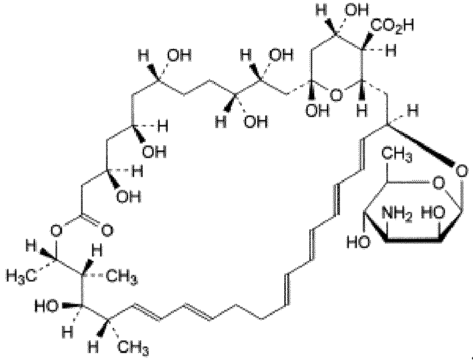

Typically, nystatin comprises hydroxyl and carboxyl groups with similar characteristics as those of amphotericin B, which enable nystatin to bond with the disclosed macro-molecule having carboxyl, amino and/or hydroxyl group to form the polyconjugate of the present invention.

In addition, natamycin (CAS No. 7681-93-8) is known to be included in Unite State Pharmacopoeia USP35-NF30, and has the following structural formula:

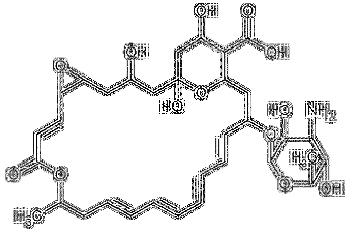

Typically, natamycin also comprises hydroxyl and carboxyl groups with similar characteristics as those of amphotericin B, which enable natamycin to bond with the disclosed macro-molecule having carboxyl, amino and/or hydroxyl group to form the polyconjugate of the present invention.

In addition, trichomycin (CAS No. 1394-02-1) is known to be included in Japanese Pharmacopoeia USP35-NF30, version XVI (JP16), and has the following structural formula:

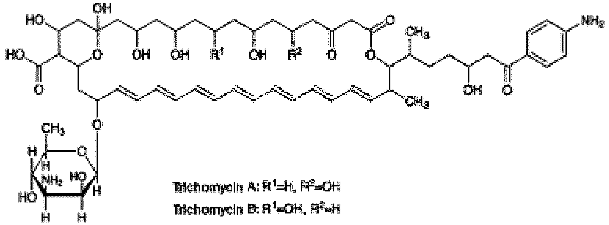

Typically, trichomycin also comprises hydroxyl and carboxyl groups with similar characteristics as those of amphotericin B, which enable trichomycin to bond with the disclosed macro-molecule having carboxyl, amino and/or hydroxyl group to form the polyconjugate of the present invention.

In addition, mepartricin (CAS No. 11121-32-7, see for example US 3780173) has the following structural formula:

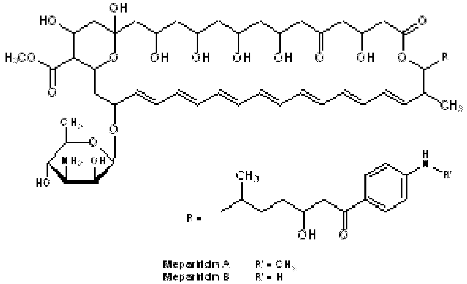

Mepartricin comprises hydroxyl groups with similar characteristics as those of amphotericin B and methyl-esterified carboxyl groups. These hydroxyl groups and the carboxyl groups esterified under certain condition enable mepartricin to bond with the disclosed macro-molecule having carboxyl, amino and/or hydroxyl group to form the polyconjugate of the present invention.

In addition, filipin III (CAS No. 480-49-9) has the following structural formula:

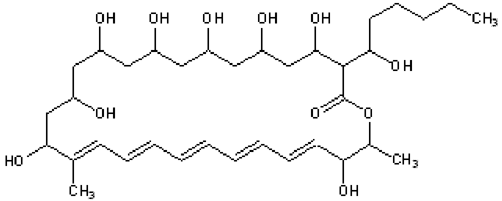

Filipin comprises hydroxyl groups with similar characteristics as those of amphotericin B. These hydroxyl groups enable filipin to bond with the disclosed macro-molecule having carboxyl group to form the polyconjugate of the present invention.

In addition, perimycin A (CAS No. 62327-61-1) has the following structural formula:

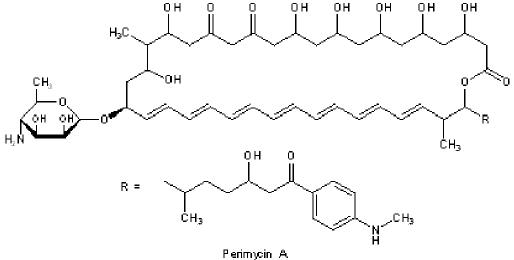

Perimycin comprises hydroxyl groups with similar characteristics as those of amphotericin B. These hydroxyl groups enable perimycin to bond with the disclosed macro-molecule having carboxyl group to form the polyconjugate of the present invention.

Other polyene macrolide compounds all have at least the hydroxyl and/or carboxyl groups with similar characteristics as those of amphotericin B, such as cannitracin (CAS No.84930-92-7, for example, the product obtained from ZHEJIANG HAIZHENG PHARM CO LTD, National Medicine Permission Number H33021810), aureofungin, etruscomycin, hamycin (CAS No. 1403-71-0) and the like. These active groups enable the polyene macrolide compounds to bond with the disclosed macro-molecule having carboxyl, amino and/or hydroxyl group to form the polyconjugate of the present invention.

In the present invention, the macro-molecule moiety can be poly-amino acid formed by polymerizing amino acids, or poly-aminohexose formed by polymerizing aminohexoses.

For poly-amino acid, the amino acid can be selected from carboxylic acid-type amino acid, amino-type amino acid, or the combination thereof, thus forming carboxylic acid-type poly-amino acid or amino-type poly-amino acid. The carboxylic acid-type amino acid is selected from aspartic acid (Asp), glutamate (Glu), or the combinations thereof at any proportion; the amino-type amino acid is selected from ornithine (Orn), lysine (Lys), arginine (Arg), hydroxylysine (Hyl), or the combinations thereof at any proportion. The amino acids can comprise D-amino acid, L-amino acid, and DL-amino acid. The method of preparing poly-amino acid is well-known to the person skilled in the art, e.g., US5610264 disclosed a method of synthesizing poly-aspartic acid, and S. Roweton et al (Journal of Environmental Polymer Degradation, Vol. 5, No.3, 1997:175) disclosed another method of synthesizing poly-aspartic acid. Similarly, the synthesis methods of other poly-amino acids have been disclosed by many references, poly-amino acids with desired degree of polymerization as well as desired molecular weight or range of molecular weight can be advantageously obtained by controlling the condition of these synthesis methods. In addition, the pharmaceutical acceptable salts of these poly-amino acids may also be used, such as sodium salt (e.g., poly-aspartic acid sodium) or hydrochloride salt (e.g., poly-ornithine) and the like. In addition, the poly-amino acid can also be purchased in the market, e.g., the poly-amino acid with the desired degree of polymerization as well as the desired molecular weight or range of molecular weight can be readily purchased from Sigma-Aldrich, for example, poly-L-aspartic acid with molecular weight of 15,000-50,000 daltons can be purchased from Sigma-Aldrich under Cat. No. P6762. In the present invention, unless otherwise specified, the poly-amino acid herein is purchased in the market.

For poly-aminohexose, the aminohexose can be selected from glucosamine, galactosamine, acetyl glucosamine, acetyl galactosamine, or the combinations thereof, thus forming poly-glucosamine, poly-galactosamine, poly-acetylglucosamine, poly-acetylgalactosamine and the like. The method of preparing poly-aminohexose is well-known to the person skilled in the art, e.g., SHAO, Jian et al (Chinese Journal of Pharmaceuticals, 1999,30(11): 481) disclosed a poly-glucosamine with specific molecular weight. The person skilled in the art can refer to the method in this reference, and advantageously obtain the poly-glucosamine with desired degree of polymerization as well as desired molecular weight or range of molecular weight by controlling the synthetic condition. In addition, the poly-glucosamine can also be purchased in the market, e.g., the poly-glucosamine with desired degree of polymerization as well as desired molecular weight or range of molecular weight can be readily purchased from Sigma-Aldrich or certain domestic companies, such as ZHEJIANG FREEMEN SHINFUDA Co., Ltd., or SHIJIAZHUANG TUOYE Co., Ltd.. In the present invention, unless otherwise specified, the poly-glucosamine herein is purchased in the market.

In one embodiment of the present invention, certain deficiencies associated with polyene macrolide substances such as amphotericin B have been overcome by forming polyconjugates between newly designed polymeric macro-molecules and polyene macrolide substances such as amphotericin B, for example, the amphotericin B polyconjugate..

In one embodiment of the polyconjugate of the present invention, one polymeric macro-molecule can bind to multiple polyene macrolide substances, such as amphotericin B.

Taking amphotericin B as an example of the polyene macrolide substance of the present invention, the provided polyconjugate can be construed as the pro-drug of amphotericin B. The conjugated amphotericin B can be gradually hydrolysed by esterase or aminoacylase to release free amphotericin B, while the polymeric macro-molecule can also break down into small molecules and be excreted from the body. The novel polyconjugate of amphotericin B, which is formed by condensing the inventive polymeric macro-molecule and amphotericin B, can also be referred to as poly-amphotericin B and the like. Poly-amphotericin B is the macro-molecule formed by the polymeric macro-molecule and amphotericin B through chemical bonds (ester bond or amide bond). In the polyconjugate, one macro-molecule carrier can bind multiple molecules of amphotericin B. The inventive poly-amphotericin B has the new characteristics of high water solubility, low toxicity/side effect, and slow releasement of amphotericin B, and therefore represents a new generation of amphotericin B. The poly-amphotericin B overcomes the deficiency of poor water solubility and high toxicity/side effect of the conventional amphotericin B. The polymeric macro-molecule carrier is non-toxic and non-allergenic, with good biocompatibility and biodegradability. Poly-amphotericin B molecule is gradually hydrolysed in vivo by esterase or aminoacylase and releases free amphotericin B, to maintain a sustained effective serum concentration and achieve a better anti-fungal therapeutic effect. The polymeric macro-molecule carrier is also broken down gradually into small molecules and be excreted from human body.

In one embodiment of the present invention, the polyconjugate of the present invention is obtained from the condensation of the carboxylic acid-type poly-amino acid with a polyene macrolide substance such as amphotericin B, e.g., by forming ester bond between the carboxyl group in the poly-amino acid and the hydroxyl group in amphotericin B. Taking the polyconjugate formed by poly-aspartic acid and amphotericin B as example, one exemplary pattern of the chemical bonds is:

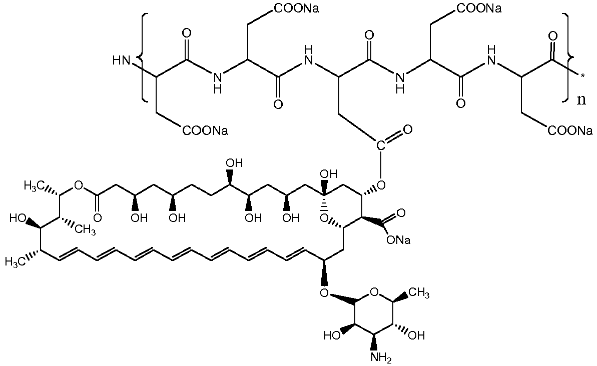

Amphotericin B can react with the carboxyl group in poly-aspartic acid in a chemical condensation reaction that uses DCC as condensing agent, through its most active hydroxyl group at the 37^{th} position (active hydroxyl), to form the ester bond (-COO-) by eliminating a molecule of H₂O. Multiple free carboxyl groups in the peptide chain of poly-aspartic acid can bind a desired amount of amphotericin B depending on the specific conditions of the condensation reaction (e.g., charging amount, reaction temperature, reaction time, etc.). For example, the weight of amphotericin B can account for 5-60% of the total weight of the polyconjugate (e.g., 10-50%, 10-40%, 10-35%). The resultant polyconjugate can form pharmaceutically acceptable salts, such as sodium salts, by reacting with basic substances. As discussed above, depending on specific needs, it is theoretically possible to condense other hydroxyly groups in amphotericin B with poly-aspartic acid to form polyconjugates by modifying the conditions of the condensation reaction, and there is no particular limitation on the site of chemical bonds in the polyconjugate. Typical examples of the above-mentioned carboxyl acid-type poly-amino acid include but not limited to D-aspartic acid (Asp), L-aspartic acid, DL-aspartic acid, D-glutamic acid (Lys), L-lysine, DL- Glutamic acid, and the combination thereof, e.g., the carboxyl acid-type poly-amino acid resulting from the polymerization of aspartic acid in any configuration with glutamic acid in any configuration at any mixing ratio such as 0-99% ratios.

In one embodiment of the present invention, the polyconjugate of the present invention is obtained from the condensation of the amino-type poly-amino acid with a polyene macrolide substance such as amphotericin B. For example, the poly-amino acid-amphotericin B is obtained from the condensation of the free amino group (-NH2) in the poly-amino acid molecule and the free carboxyl group (-COOH) in amphotericin B molecule, with elimination of one molecule of water in the presence of a catalyst. Taking the polyconjugate formed by poly-ornithine and amphotericin B as example, one exemplary pattern of the chemical bonds is:

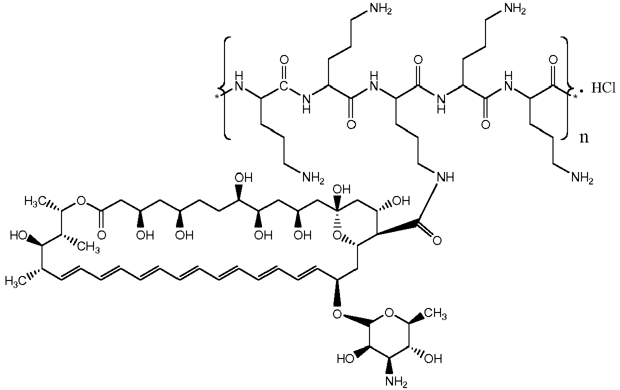

Similar to the abovementioned poly-aspartic acid-amphotericin B polyconjugate, multiple free amino groups in the poly-ornithine chain can bind a desired amount of amphotericin B depending on the specific conditions of the condensation reaction (e.g., charging amount, reaction temperature, reaction time, etc.). Similarly, the polyconjugate is proved to result from chemical bonding rather than physical bonding (e.g. hydrogen bond) hereinafter by ultra-violet spectroscopy and HPLC, there is no particular limitation on the site of chemical bonds in the polyconjugate. Examples of the typical amino-type poly-amino acid include but not limited to D-ornithine (Orn), L-ornithine, DL-ornithine, D-lysine (Lys), L-lysine, DL-lysine, D-argnine (Arg), L-argine, DL-argnine, D-hydrolysine (Hyl), L-hydrolysine, DL-hydrolysine, and the combination thereof, e.g., the amino-type poly-amino acid result from the polymerization of ornithine in any configuration with lysine in any configuration at any mixing ratio such as 1-99% ratios.

In one embodiment of the present invention, the polyconjugate of the present invention is obtained from the condensation of the poly-aminohexose with a polyene macrolide substance such as amphotericin B. For example, the poly-aminohexose-amphotericin B is obtained from the condensation of the free hydroxyl group (-OH) in the poly-aminohexose (poly-glucosamine) molecule and the free carboxyl group (-COOH) in amphotericin B molecule, with elimination of one molecule of water in the presence of a catalyst and formation of ester bond (-COO-). Taking the polyconjugate formed by poly-glucosamine and amphotericin B as example, one exemplary pattern of the chemical bonds is:

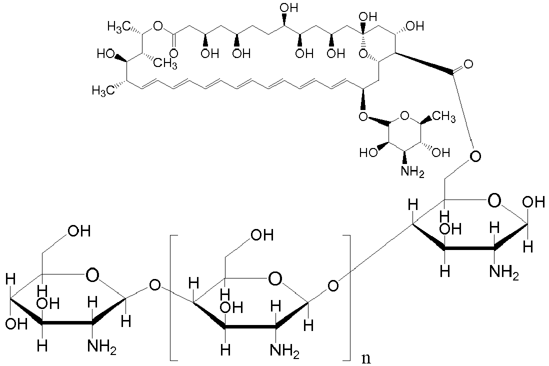

Similar to the abovementioned poly-aspartic acid-amphotericin B polyconjugate, multiple free hydroxyl groups in the poly-glucosamine chain can bind a desired amount of amphotericin B depending on the specific conditions of the condensation reaction (e.g., charging amount, reaction temperature, reaction time, etc.). Similarly, the polyconjugate is proved to result from chemical bonding rather than physical bonding (e.g. hydrogen bond) hereinafter by ultra-violet spectroscopy and HPLC, there is no particular limitation on the site of chemical bonds in the polyconjugate. Examples of a typical poly-aminohexose include but not limited to poly-glucosamine, poly-galactosamine, poly-acetyl glucosamine, poly-acetyl galactosamine and the like.

In the process of synthesizing a compound of the present invention, all the used raw materials can be prepared by a skilled person according to prior art, or prepared according to methods known in the prior art, or commercially available. The intermediates, raw materials, reagents and reaction conditions used in the above reaction scheme can all be properly modified by those skilled in the art.

The polyconjugate of the present invention can be used in combination with other active ingredients, as long as such combination use does not produce additional adverse effect, such as anaphylaxis.

The polyconjugate of the present invention can be used as a drug alone, or in combination with one or more additional drugs which have synergistic and /or additive effect with the invented polyconjugates. The combined therapy is carried out by administering each of therapeutic components simultaneously, sequentially or separately.

In the present invention, the term "pharmaceutical composition" refers to a product comprising the designated amounts of the designated ingredients, and any products directly or indirectly obtained by combining various designated ingredients of designated amounts. The term "pharmaceutical composition" as used herein is used interchangeably with the term "composition".

The actual dose level of various active ingredients in a pharmaceutical composition of the present invention can vary so that the desired therapeutic effects can be achieved depending on specific patients, compounds and administration modes. The dose level must be determined according to the activity of specific compound, administration route, severity of disease to be treated, and conditions and medical history of patients. However, the conventional method in the art is to increase gradually the dose of compound from a level lower than that for achieving desired therapeutic effects to a level enough to achieve the desired therapeutic effects.

In the aforementioned or other treatment and/or prophylaxis, a polyconjugate of the present invention in a therapeutically and/or prophylactically effective amount can be used in form of pure compound, or in form of pharmaceutical composition comprising the polyconjugate disclosed herein and one or more pharmaceutically acceptable excipients. The phrase "therapeutically and/or prophylactically effective amount" of the polyconjugate of the present invention means that the polyconjugate is in an amount sufficient to achieve prophylactic and/or therapeutic effects with a reasonable benefit to risk ratio. It should be understood that the total amount per day of the polyconjugate or pharmaceutical composition of the present invention must be determined by a physician within the range of reliable medical decisions. As for any specific patients, the specific therapeutic amount must be determined based on various factors, including the diseases to be treated and severity thereof, the activity of the specific polyconjugate to be used, the specific pharmaceutical composition to be used, age, gender, body weight, general health status, and food of patient; the administration time and route and clearance rate of the specific pharmaceutical composition to be used, co-medication/the drug(s) administered in combination or simultaneously with the specific pharmaceutical composition, co-medication, as well as other similar factors well known in the art of medicine. For example, it is a common method in the art to increase gradually the dose of compound from a level lower than that for achieving desired therapeutic effects to a level enough to achieve the desired therapeutic effects. In general, the dose of a polyconjugate of the present invention for a mammal especially a human being can be 0.0001-1000 mg/kg body weight per day, such as 0.001-500 mg/kg body weight per day, 0.001-100 mg/kg body weight per day.

A pharmaceutical composition comprising an effective amount of the polyconjugate of the present invention can be prepared by using a drug carrier well-known by those skilled in the art. Hence, the present invention further provides a pharmaceutical composition comprising the polyconjugate of the present invention formulated with one or more non-toxic pharmaceutically acceptable carrier. The pharmaceutical composition can be specifically formulated in solid or liquid form for oral administration, parenteral injection or rectal administration.

The pharmaceutical composition can be formulated in many dosage forms for facilitating administration, for example, oral preparations (such as tablets, capsules, solutions or suspensions); injectable preparations (such as injectable solutions or suspensions, or injectable dry powders that can be immediately used by adding water before injection). The carrier in the pharmaceutical composition comprises for oral preparations: binders (such as starch, typically being starches of corn, wheat or rice, gelatin, methylcellulose, sodium carboxymethylcellulose and/or polyvinylpyrrolidone), diluents, lubricants (such as silicon dioxide, talc, stearic acid or salts thereof, typically being magnesium stearate or calcium stearate, and/or polyethylene glycol), if desired, further comprises disintegrating agents such as starch, agar, alginic acid or salts thereof, typically sodium alginate, and/or effervescence mixtures, co-solvents, stabilizing agents, suspending agents, coloring agent, flavoring agent, etc.; for injectable preparations: preservatives, solubilizing agents, stabilizing agents etc.; for topical preparations: substrates, diluents, lubricants, etc. The pharmaceutical preparations can be administered orally or parenterally (such as intravenously, subcutaneously or topically), and if some drugs are not stable in gastral conditions, they can be formulated as enteric coated tablets.

More specifically, the pharmaceutical composition of the present invention can be administered orally, rectally, parenterally, endoluminally, endovaginally, intraperitoneally, topically (such as via powder, ointment or drops), buccally to a human or other mammal, or administered as oral spray or nasal spray. The term "parenteral" used herein refers to administration manners including intravenous, intramuscular, intraperitoneal, intrathoracic, subcutaneous and intraarticular injection or transfusion.

The pharmaceutical composition suitable for parenteral injection can comprise physiologically acceptable sterile aqueous or nonaqueous solvent, dispersant, suspending agent, or emulsifying agent, as well as sterile dispersant for reforming a sterile injectable solution or dispersion. The examples of suitable aqueous or nonaqueous carriers, diluents, solvents or media include water, ethanol, polyols (propylene glycol, polyethylene glycol, glycerol, etc.), vegetable oil (such as olive oil), injectable organic esters such as ethyl oleate and suitable mixtures thereof

These pharmaceutical compositions can further comprise excipients, such as preservative, wetting agent, emulsifying agent and dispersant. The use of various antibacterial agents and antifungal agents, such as nipagins, nautisan, phenol, sorbic acid, etc. can ensure effects of combating microorganisms. It is also desired to comprise isotonizing agents such as sugars, sodium chloride, etc. Prolonged absorption of the injectable pharmaceutical form can be brought about by the use of agents delaying absorption, for example, aluminum monostearate and gelatin.

Besides the active compound, the suspension can further comprise a suspending agent, such as ethoxylated isooctadecanol, polyoxyethylene sorbitol and polyoxyethylene sorbitan, microcrystalline cellulose, meta-aluminum hydroxide, bentonite, agar and tragacanth gum, or mixtures of these substances.

In some cases, it is desired to reduce the absorption rate of subcutaneously or intramuscularly administered drug for prolonging the effect of drug. This can be realized by using a liquid suspension of crystal or amorphous form with poor water solubility. Thus, the absorption rate of drug depends on its dissolution rate, while the dissolution rate depends on the size and form of crystal. Or, the delayed absorption of drug in parenteral administration can be reached by dissolving or dispersing the drug in an oil medium.

An injectable depot dosage form can be prepared by forming microcapsule substrate of drug in a biodegradable polymer such as polylactide-polyglycolide. The release rate of drug can be controlled according to the ratio of drug to polymer and the properties of the specifically used polymer. Other examples of biodegradable polymer comprise poly (orthoesters) and poly (anhydrides). The injectable depot dosage form can also be prepared by embedding drug in a liposome or microemulsion compatible to body tissues.

The injectable preparation can be sterilized by filtration using a bacterial-removing filter or by incorporating a sterilizing agent in form of sterile solid composition, and the solid composition can be dissolved or dispersed in sterile water or other sterile injectable media before clinical application.

The compound of the present invention or pharmaceutical composition thereof can be administered orally or parenterally. Those for oral administration can be tablets, capsules, coated dosage form, and pharmaceutical preparations for parenteral administration can be injections and suppository. These preparations are prepared according to methods well-known by those skilled in the art. In order to manufacture tablets, capsules and coated dosage forms, the excipients used are commonly used excipients, such as starch, gelatin, arabic gum, silica, polyethylene glycol, the solvents used for liquid dosage forms are water, ethanol, propylene glycol, vegetable oils (such as corn oil, peanut oil, oliver oil, etc.). The preparations comprising the compound of the present invention can further comprise other auxiliary materials, such as surfactants, lubricants, disintegrants, preservatives, flavoring agent and coloring agent, etc.. In tablets, capsules, coated dosage forms, injections and suppositories, the dose of the polyconjugste of the present invention is expressed in an amount of the polyene macrolides existed in unit dosage form. In unit dosage form, the amount of the polyene macrolide compound of the present invention usually is 0.01-5000 mg, a preferable unit dosage form contains 0.1-500 mg, a more preferable unit dosage form contains 1-500mg. Specifically, the solid dosage form for oral administration as provided in the present invention comprise capsules, tablets, pills, powders and granules. In such solid dosage forms, the active compound can be mixed with at least one inert pharmaceutically acceptable excipient or carrier such as sodium citrate or dicalcium phosphate and/or the following substances: a) filler or bulking agent,; b) binding agent, such as carboxymethyl cellulose, alginate, gelatin, polyvinyl pyrrolidone, sucrose, and arabic gum; c) humectant, such as glycerol; d) disintegrating agent, such as agar, calcium carbonate, potato or cassava starch, alginic acid, some silicates and sodium carbonate; e) solution blocking agent, such as paraffin wax; f) absorption accelerator, such as quaternary ammonium compounds; g) wetting agent, such as cetanol and glycerol monostearate; h) adsorbent, such as kaolin and bentonite; and i) lubricant, such as talc, calcium stearate, magnesium stearate, solid polyethylene glycol, sodium dodecylsulfate and their mixtures. In the cases of capsules, tablets and pills, these dosage forms may also comprise a buffering agent.

A solid composition of similar type uses excipients such as lactose and high molecular weight polyethylene glycol which can also be used as fillers of soft capsules and hard capsules.

The solid dosage forms of tablets, dragees, capsules, pills and granules can be prepared with coating agents and shell materials such as enteric coating materials and other coating materials well-known in the field of medical preparations. These solid dosage forms can optionally comprise sun-screening agent, and their composition can allow they merely or preferentially release active ingredient at some sites of intestinal tract optionally in a delayed manner. Examples of the potential embedding composition comprise high molecular materials and waxes. If appropriate, the active substance can be formulated in form of microcapsules with one or more aforementioned excipients.

The liquid dosage form for oral administration comprises pharmaceutically acceptable emulsifying agent, solvent, suspending agent, syrup and elixir. Besides the active compound, the liquid dosage form may further comprise an inert diluent commonly used in the art, such as water or other solvent, solubilizer and emulsifying agent, such as ethanol, isopropanol, ethyl carbonate, ethyl acetate, benzyl alcohol, benzyl benzoate, propylene glycol, butane-1,3-diol, dimethyl formamide, oils (such as cottonseed oil, peanut oil, corn oil, embryo oil, olive oil, castor oil, and sesame oil), glycerol, tetrahydrofurfuryl alcohol, fatty acid esters of polyethylene glycol and sorbitan, and the mixtures thereof Besides inert diluents, the compositions for oral administration can further comprise auxiliary materials, such as wetting agents, emulsifying agents and suspending agents, sweetening agents, flavoring agent and flavors.

The composition for rectal or vaginal administration is preferably a suppository. The suppository can be prepared by mixing the polyconjugate of the present invention with a suitable non-irritative excipient or carrier, such as cocoa butter, polyethylene glycol or suppository wax, they can be solid at room temperature, but liquid at body temperature, and can release an active compound in rectal lumen or vaginal canal.

It is also desired to use the polyconjugate of the present invention for topical administration. The dosage form of the polyconjugate of the present invention for topical administration comprises powder, spray, ointment and inhalation. The active substance and a pharmaceutically acceptable carrier can be mixed under sterile conditions with any desired preservative, buffering agent or propellant. Ophthalmic preparation, eye salve, powder and solution are all in the scope of the present invention.

The polyconjugate of the present invention can be administered in a form of liposome. It is well known in the art, liposome usually is prepared by using phospholipid or other lipids. Liposome is formed with monolayer or multilayer hydrated liquid crystal which is dispersed in an aqueous medium. Any non-toxic, physiologically acceptable and metabolizable lipids capable of forming liposome can be usable. The compound of the present invention in liposome form can comprise stabilizing agent, preservative, excipient, besides the compound of the present invention. Preferable lipids are natural and synthetic phospholipids and phosphatidylcholines (lecithin), they can be used solely or together. The methods for forming liposome are well-known in the art. References can be seen, for example, Prescott, Ed., Methods in Cell Biology, Volume XIV, Academic Press, New York, N.Y. (1976), p. 33.

The inventors of the present application have surprisingly found that the polyconjugate of the present invention shows encouraging beneficial effects in biological and/or physical and/or chemical aspects.

### DESCRIPTION OF THE FIGURES

Figure 1 is the ultraviolet-visible absorption spectra of amphotericin B.
Figure 2 is the ultraviolet-visible absorption spectra of poly-aspartic acid.
Figure 3 is the ultraviolet-visible absorption spectra of poly-aspartic acid-amphotericin B polyconjugate (PA-AB).
Figure 4 is the high performance liquid chromatogram of amphotericin B (AB, 28 µg/ml).
Figure 5 is the high performance liquid chromatogram of poly-aspartic acid (used in Preparation 1, PA, 80 µg/ml).
Figure 6 is the high performance liquid chromatogram of poly-aspartic acid-amphotericin B polyconjugate.
Figure 7 is the high performance liquid chromatogram of the physical mixture of poly-aspartic acid-amphotericin B polyconjugate and free amphotericin B.
Figure 8 is the hydrogen-nuclear magnetic resonance (1H-NMR) spectrum of poly-aspartic acid-amphotericin B polyconjugate (PA-AB).
Figure 9 is the carbon-nuclear magnetic resonance (13C-NMR) spectrum of poly-aspartic acid-amphotericin B polyconjugate (PA-AB).
Figure 10 shows the atlas of the high performance liquid chromatography (HPLC) separation of poly-aspartic acid-amphotericin B polyconjugate (PA-AB) with ultraviolet (UV) detection.
Figure 11 shows the atlas of the HPLC separation of poly-aspartic acid-amphotericin B polyconjugate (PA-AB) with mass spectrometry (MS).
Figure 12 shows the atlas of the HPLC separation of amphotericin B (AB) with UV detection.
Figure 13 shows the atlas of the HPLC separation of amphotericin B (AB) with mass spectrometry (MS).

### EXAMPLES

The invention is further described by reference to the following examples of preparation and experiments. However, it should be understood that these examples are provided for purposes of illustration only, and are not intended to limit the scope of the invention in any way.

The materials and methods used in examples are generally and/or specifically described in the present invention. Although many materials and operation methods used for fulfilling the purpose of the present invention are known in the art, they are still described as detailed as possible. Those skilled in the art clearly know that if not described particularly, the materials and methods used in the present invention are well known in the art.

### A. Methods for testing polyconjugate

### Method 1: Ultraviolet-Visible Spectrophotometry (UV -VIS)

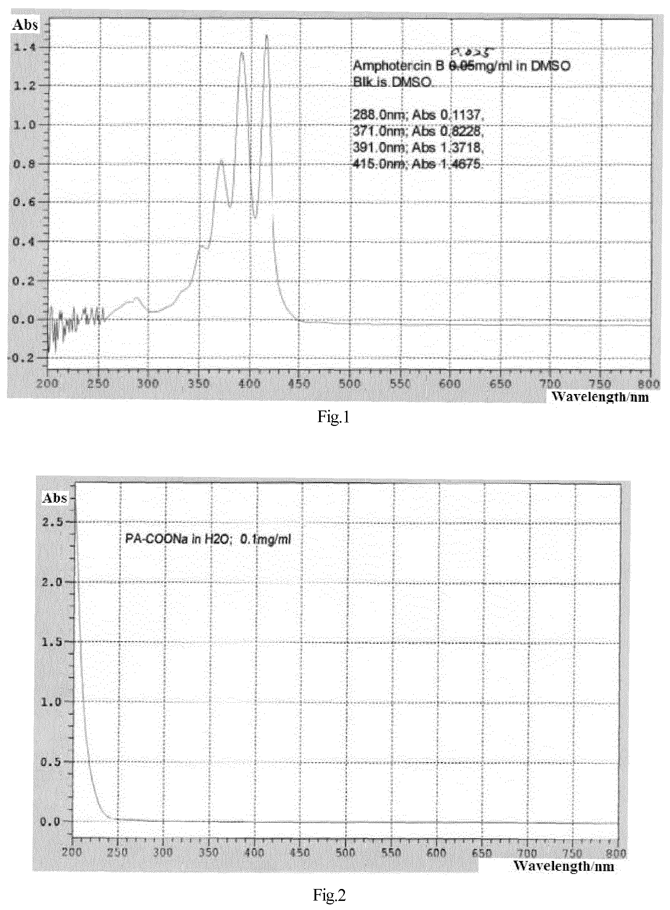

Amphotericin B (25 µg/mL dissolved in DMSO, DMSO as blank control) was analyzed by ultraviolet-visible (UV-VIS) spectrophotometry, as shown in Fig. 1. The figure shows the lack of absorption at wavelength shorter than 300 nm or longer than 500 nm, and the presence of absorption at wavelength between 350 nm and 450 nm, particularly the typical absorption peaks at 371 nm, 391 nm, and 415 nm.

Poly-aspartic acid (used in Preparation 1, 100 µg/mL dissolved in water) was analyzed by UV-VIS spectrophotometry, as shown in Fig. 2. The figure shows the lack of absorption at wavelength longer than 250 nm, and the presence of typical end absorption at wavelength shorter than 250 nm, suggesting that poly-aspartic acid does not have contribution to the absorption value of the polyconjugate or amphotericin B at wavelength longer than 250 nm.

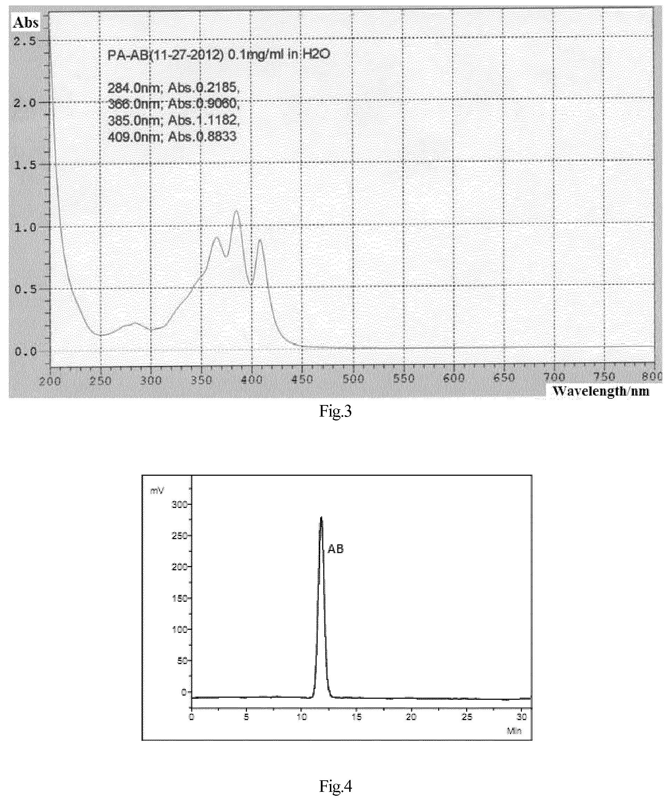

Poly-aspartic acid-amphotericin B polyconjugate (PA-AB) (used in Preparation 1, 100 µg/ml, dissolved in water) was analyzed by UV-VIS spectrophotometry, as shown in Fig. 3. The figure shows the lack of absorption at wavelength longer than 500 nm, the presence of typical end absorption at wavelength shorter than 250 nm (contributed by poly-aspartic acid), and the presence of absorption at wavelength between 350 nm and 450 nm (primarily contributed by amphotericin B), particularly the typical absorption peaks at 366 nm, 385 nm, and 409 nm. Possibly due to the formation of chemical bonds in the polyconjugate, these three typical absorption peaks are shifted a little compared to the wavelengths of the corresponding peaks shown in Fig. 1, but still provide the typical absorption spectra of amphotericin B as a whole.

Figures 1 to 3 demonstrated that in the spectrophotometric measurement, the three substances, poly-aspartic acid, amphotericin B and the polyconjugate thereof, all have distinctive and independent spectral behaviors and spectral contributions characterized by their UV-VIS absorption spectra, which enable to analyze the three substances qualitatively and quantitatively by HPLC method.

In addition, the inventors have found by further experiments that the UV-VIS absorption spectra of other poly-amino acids, poly-aminohexoses, polyene macrolide antibiotics as well as poly-conjugates consist of the poly-amino acids or poly-aminohexoses and polyene macrolide antibiotics all display the characteristics similar to those of the above UV-VIS absorption spectra obtained from poly-aspartic acid, amphotericin B and the polyconjugate thereof The distinctive and independent spectral behaviors and spectral contributions displayed by these three classes of substances in spectrophotometric analysis enable to analyze them qualitatively and quantitatively by HPLC method. For example, poly-ornithine, poly-glucosamine and poly-glutamate are similar to poly-aspartic acid, and would not influence/interfere the spectral characteristics of amphotericin B. In another example, the spectral characteristics of natamycin were not influenced or interfered by poly-aspartic acid, poly-ornithine, poly-glutamate, poly-glucosamine, poly-galactosamine and the like.

### Method 2: High Performance Liquid Chromatography (HPLC)

According to the methods described under the title of "detection of substances related to amphotericin B" in Chinese Pharmacopoeia 2010, Part II, page 328, the condition was: C18 chromatographic column (4.6*30mm, 5µm), mobile phase: acetonitrile-methanol-0.05mol/L ammonium acetate containing 0.073% ethylenediamine tetraacetic acid (pH value is adjusted by glacial acetic acid to 5.0) (260:450:320), flow rate: 1.0 ml/min, detection wavelength: 405 nm. Figure 4 is the high performance liquid chromatogram of amphotericin B (AB, 28 µg/ml), wherein the first peak of amphotericin B appeared at about 12 minutes.

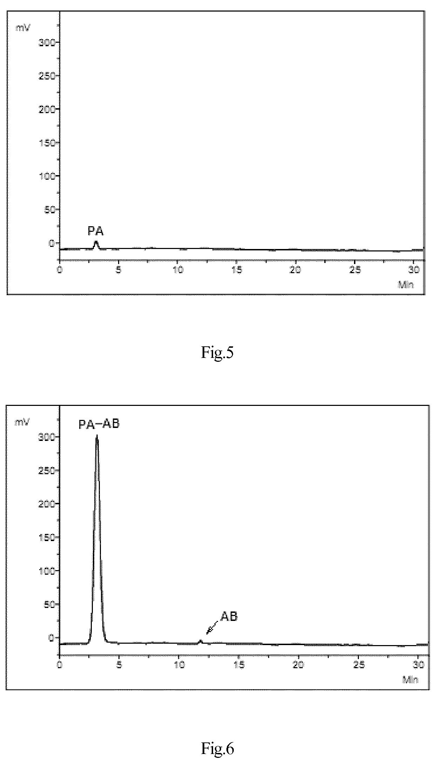

The high performance liquid chromatogram of poly-aspartic acid was obtained according to the above HPLC method. As shown in figure 5, a small chromatographic peak at about 3 min is brought about by the weak absorption of PA at 405 nm detection wavelength.

The high performance liquid chromatogram of poly-aspartic acid-amphotericin B polyconjugate (PA-AB, product of Preparation 1, 105 µg/ml, containing amphotericin B in a concentration comparable to that of the sample solution used in figure 4) was obtained according to the above HPLC method. As shown in figure 6, no chromatographic peak was detected at about 12 min (i.e., no free AB), but the chromatographic peak at about 3 min had peak area comparable with that in figure 4. It has demonstrated that the method of the present invention can produce PA-AB polyconjugate with high purity (purity higher than 99% based on the weight of AB), which means the polyconjugate contains essentially no free AB (the peak area percentage is lower than 1% by counting with area normalization). PA-AB is a stable compound formed through chemical bonds and will not break apart under chromatographic conditions. Because of the strong polarity of the poly-amino acid, the polyconjugate of PA-AB has the chromatographic behavior similar to PA and the peak at the same time as PA. In addition, the percentage of AB in the polyconjugate, i.e., the active drug content, can be calculated by combining the results of the HPLC method and the measurement of figure 4. The active drug content of preparation 1 is 23.4% (w/w).

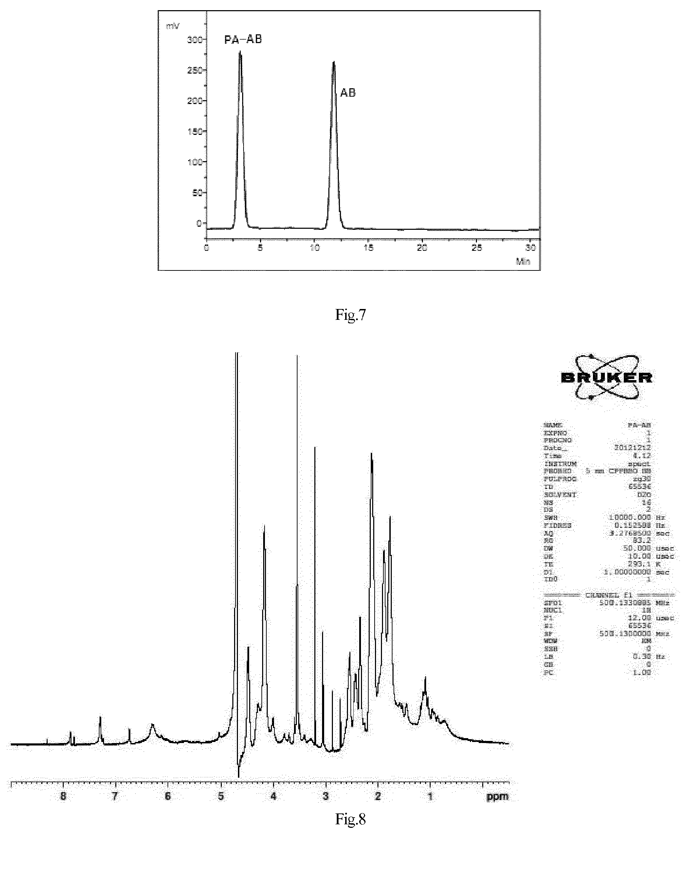

The high performance liquid chromatogram of the physical mixture of poly-aspartic acid-amphotericin B polyconjugate and free amphotericin B (PA-AB, 100 µg/ml; AB, 26 µg/ml) was obtained according to the above HPLC method. As shown in figure 7, a chromatographic peak representing AB was detected at about 12 min with the peak area corresponding to the added quantity of AB, and a PA-AB peak was detected at about 3 min corresponding to the added quantity of PA-AB. It has demonstrated that the chromatographic behaviors of the PA-AB polyconjugate and the free AB are independent and will not interfere with each other.

The HPLC method can also be adapted to analyze the polyconjugate consist of amphotericin B and other macro-molecular polymer. Without major modifications of the HPLC conditions, the method can be used for analyzing these polyconjugate qualitatively or quantitatively.

The HPLC method can also be adapted to analyze polyconjugates consist of other polyene macrolide antibiotics and macro-molecular polymer. With proper modification of the HPLC conditions such as the mobile phase, the method can be used for analyzing these polyconjugates qualitatively or quantitatively. HPLC method and conditions used for other polyene macrolide compounds which are documented in other drug standards and refereneces can be used a reference; e.g., trichomycin, whose HPLC method and conditions can be found in JP16.

### B. Preparations of Polyconjugate

### Preparation 1. chemical synthesis of poly-aspartic acid-amphotericin B polyconjugate

The synthetic process is schematically expressed by the following scheme:

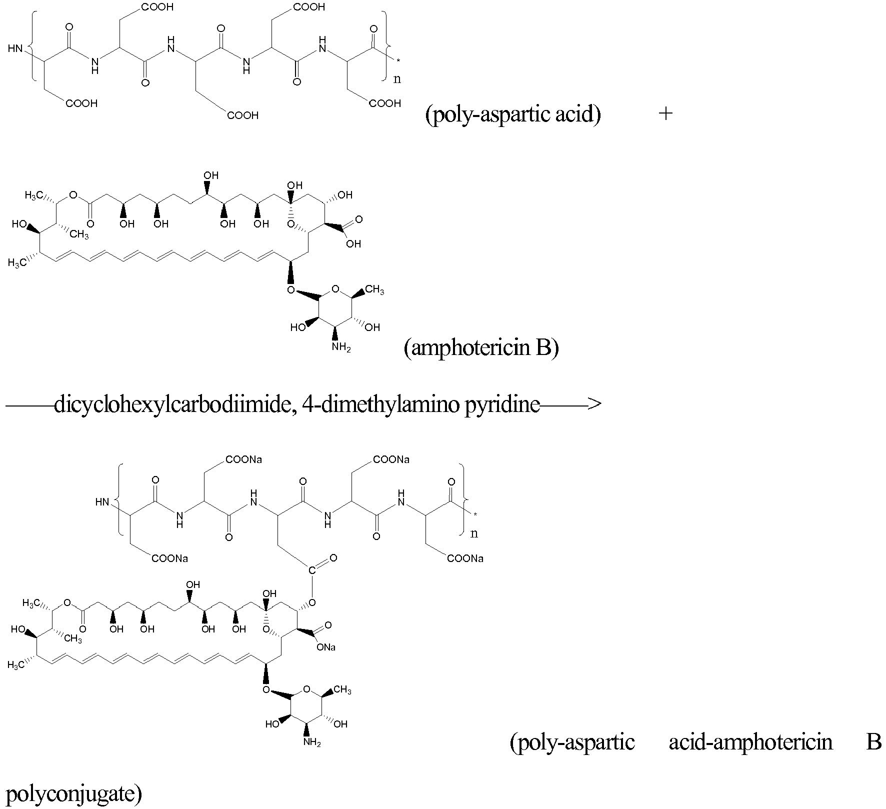

Synthetic steps: Dissolving poly-L-aspartic acid 800.0 mg (molecular weight 28,000 Da) in 5 ml dimethyl formamide. Adding amphotericin B 280.0 mg (0.30 mol) into the dimethyl formamide solution, stirring and dissolving. Adding dicyclohexylcarbodiimide 62.5 mg (0.30 mol) and 4-dimethylamino pyridine 37.0 mg (0.30 mol) into the dimethyl formamide solution, magnetically stirring for 12 hours under room temperature (23-25°C). Adding 100 ml 1M NaHCO₃ into the reaction solution, magnetically stirring for 1 hour, transferring this basic solution into a dialysis bag, dialyzing against deionized water for 5 hours, passing through a 0.2µ filter, and freeze dried. 1,033.4 mg poly-aspartic acid-amphotericin B polyconjugate was obtained.

Figure 8 is the hydrogen-nuclear magnetic resonance (¹H-NMR) spectrum of poly-aspartic acid-amphotericin B polyconjugate obtained from this preparation, showing a spectral line coincides with that of the polyconjugate.

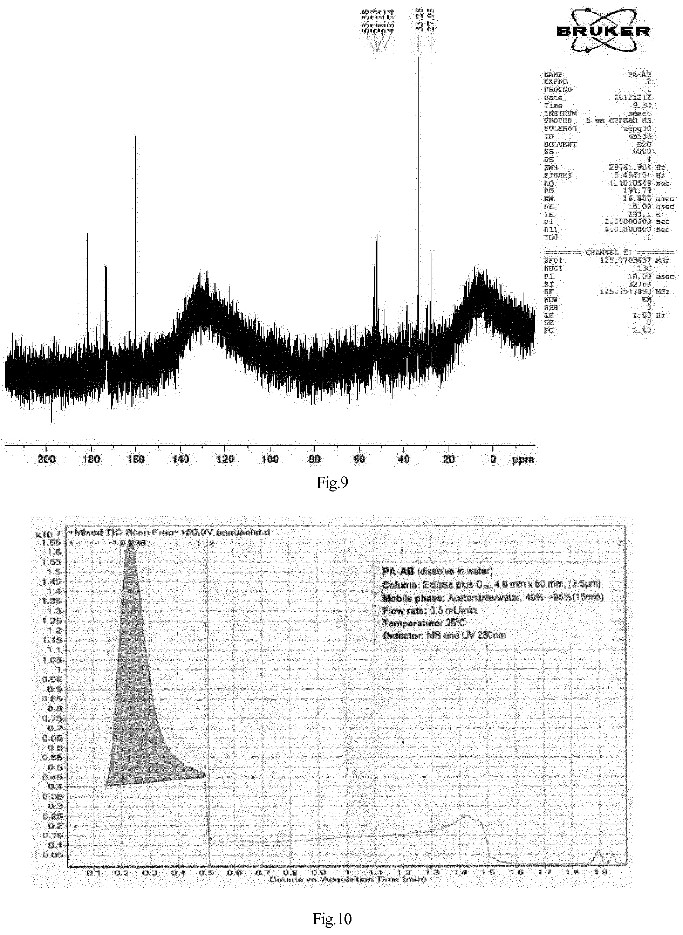

Figure 9 is the carbon-nuclear magnetic resonance (¹³C-NMR) spectrum of poly-aspartic acid-amphotericin B polyconjugate obtained from this preparation, showing a spectral line coincides with that of the polyconjugate.

Figure 10 is the spectrum of the high performance liquid chromatography (HPLC) separation of poly-aspartic acid-amphotericin B polyconjugate obtained from this preparation with ultraviolet (UV) detection.

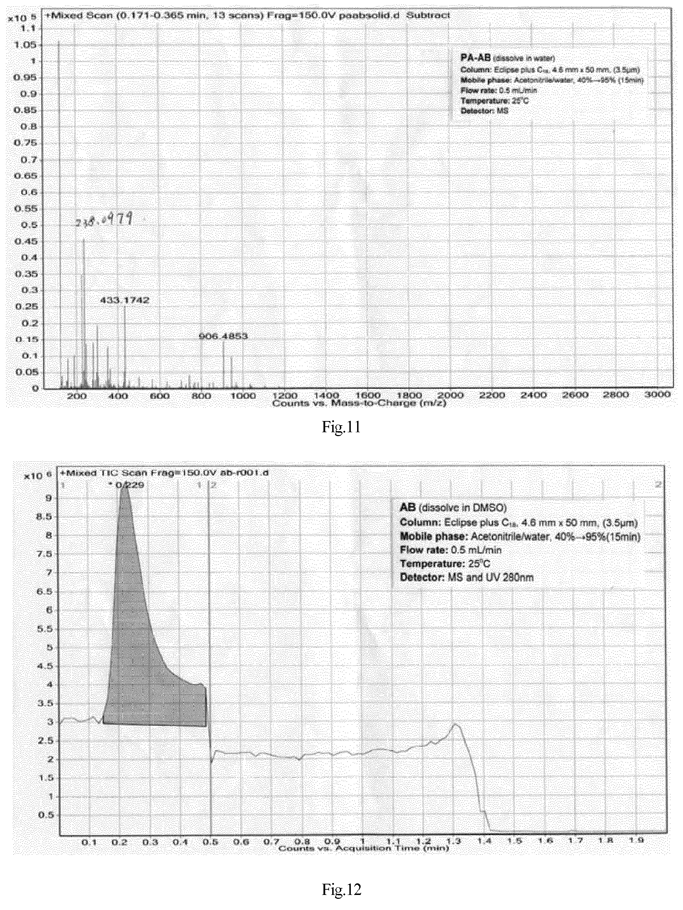

Figure 11 is the spectrum of the HPLC separation under the same HPLC conditions as those used in Fig. 10, of poly-aspartic acid-amphotericin B polyconjugate obtained from this preparation with mass spectrometry (MS). The figure shows several fragments formed by amphotericin B, indicating the presence of amphotericin B in this polyconjugate and it was eluted with the macro-molecular polymer together in the HPLC system.

Figure 12 is the spectrum of the HPLC separation under another set of test condition, of poly-aspartic acid-amphotericin B polyconjugate obtained from this preparation with UV detection.

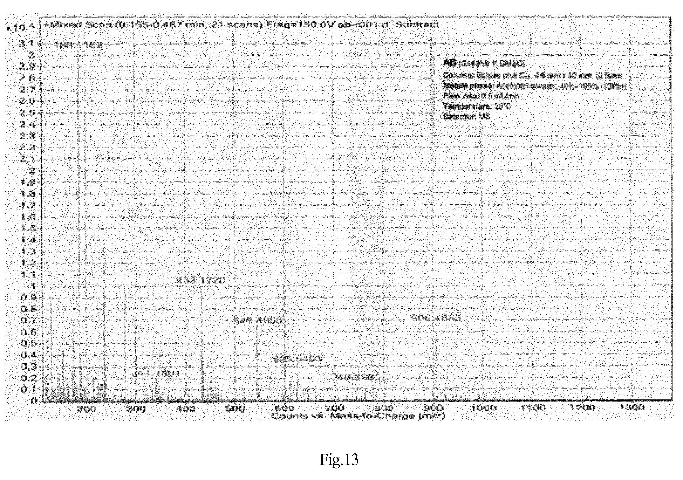

Figure 13 is the spectrum of the HPLC separation under the same HPLC conditions as those used in figure 12, of poly-aspartic acid-amphotericin B polyconjugate obtained from this preparation with mass spectrometry (MS). The figure shows several fragments formed by amphotericin B, indicating the presence of amphotericin B in this polyconjugate and it was eluted with the macro-molecular polymer together in the HPLC system.

According to the experimental data, the active drug content = 23.4% (w/w), the free active drug content < 0.2% (calculated by dividing the peak area of AB peak in Fig. 6 by the area of peak PA-AB and multiplying by 100%).

### Preparation 2. chemical synthesis of poly-ornithine-amphotericin B polyconjugate

The synthetic process is schematically expressed by the following scheme:

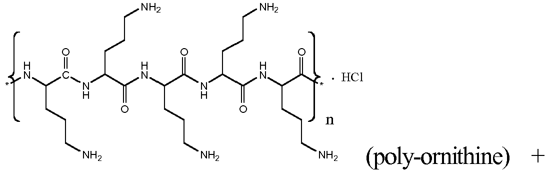

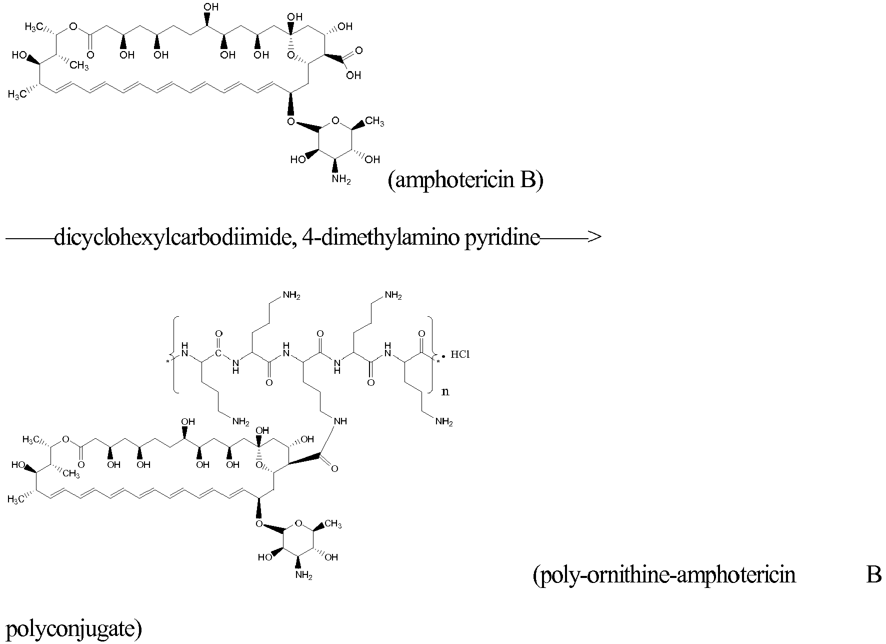

Synthetic steps: Dissolving poly-D-ornithine 250.0 mg (molecular weight 34,000 Da) in 3 ml dimethyl formamide. Adding amphotericin B 75.0 mg (0.08 mol) into the dimethyl formamide solution, and dissolving. Adding dicyclohexylcarbodiimide 16.7 mg (0.08 mol) and 4-dimethylamino pyridine 10.0 mg (0.08 mol) into the dimethyl formamide solution, magnetically stirring for 12 hours under room temperature (23-25°C). Adding 50 ml water into the reaction solution, transferring this solution into a dialysis bag, dialyzing against deionized water for 5 hours, passing through a 0.2µ filter, and freeze dried. 323.0 mg poly-ornithine-amphotericin B polyconjugate was obtained.

The resultant polyconjugate formed by chemical bonds was confirmed by the methods of UV-VIS spectroscopy, HPLC, hydrogen-nuclear magnetic resonance (¹H-NMR), carbon-nuclear magnetic resonance (¹³C-NMR), HPLC-UV, and HPLC-MS which are used for validating the product of preparation 1.

According to the experimental data, the active drug content = 20.8% (w/w), the free active drug content is 0% (undetected).

### Preparation 3. chemical synthesis of poly-glucosamine-amphotericin B polyconjugate

The synthetic process is schematically expressed by the following scheme:

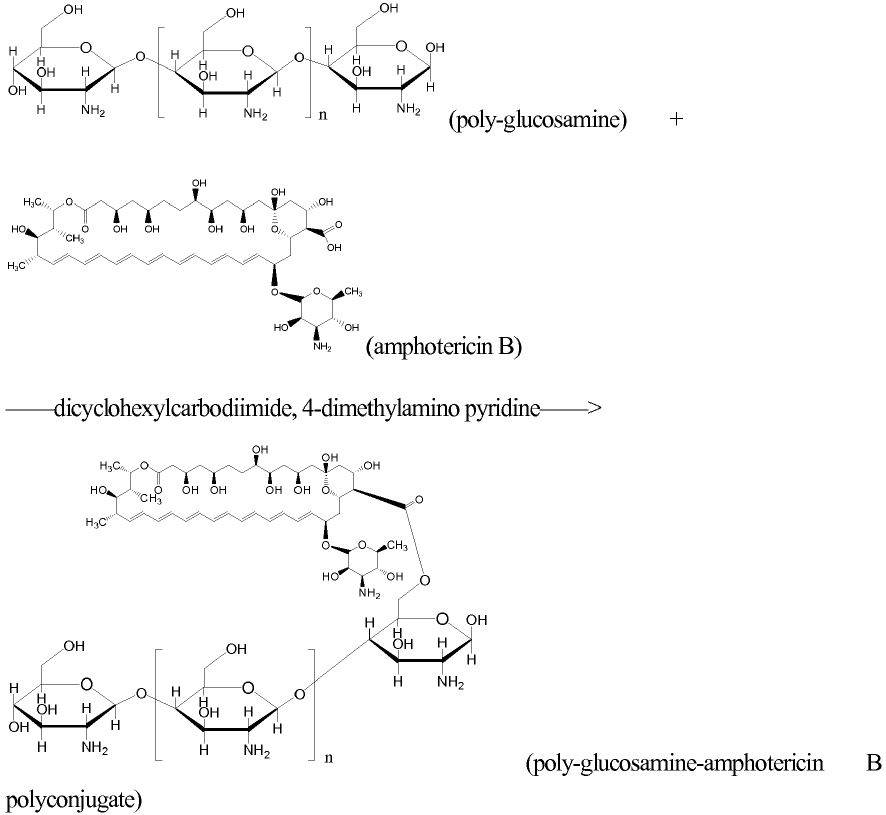

Synthetic steps: Dissolving poly-glucosamine 532.0 mg (molecular weight 85,000 Da) in 6 ml dimethyl formamide. Adding amphotericin B 305.0 mg (0.33 mol) into the dimethyl formamide solution, and dissolving. Adding dicyclohexylcarbodiimide 68.1 mg (0.33 mol) and 4-dimethylamino pyridine 40.3 mg (0.33 mol) into the dimethyl formamide solution, magnetically stirring for 12 hours under room temperature (23-25°C). Adding 80 ml 0.1 M HCl into the reaction solution, transferring this acidic solution into a dialysis bag, dialyzing against deionized water for 5 hours, passing through a 0.2µ filter, and freeze dried. 803.5 mg poly-glucosamine-amphotericin B polyconjugate was obtained.

The resultant polyconjugate formed by chemical bonds was confirmed by the methods of UV-VIS spectroscopy, HPLC, hydrogen-nuclear magnetic resonance (¹H-NMR), carbon-nuclear magnetic resonance (¹³C-NMR), HPLC-UV, and HPLC-MS which are used for validating the product of preparation 1.

According to the experimental data, in the resultant product, the active drug content = 33.6% (w/w), the free active drug content <0.4%.

### Preparation 4. chemical synthesis of macro-molecule-amphotericin B polyconjugate

The method referred to the method used in preparation 1, but instead poly-DL-aspartic acid 1,400.0 mg (molecular weight 15,000 Da) was used as the macro-molecular polymer. 1,511.5 mg polyconjugate was obtained. According to the experimental data, in the resultant product, the active drug content = 15.7% (w/w), the free active drug content <0.8%.

### Preparation 5. chemical synthesis of macro-molecular polymer-amphotericin B polyconjugate

The method referred to the method used in preparation 1, but instead poly-(L-ornithine/L-lysine) 1,000.0 mg (molar ratio 1:1) (molecular weight 25,000 Da) was used as the macro-molecular polymer. 1,191 mg polyconjugate was obtained. According to the experimental data, in the resultant product, the active drug content = 19.8% (w/w), the free active drug content is 0% (undetected).

### Preparation 6. chemical synthesis of macro-molecular polymer-amphotericin B polyconjugate

The method referred to the method used in preparation 3, but instead poly-galactosamine (molecular weight 150,000 Da) was used as the macro-molecular polymer. 811 mg polyconjugate was obtained. According to the experimental data, in the resultant product, the active drug content = 23.5% (w/w), the free active drug content <0.3%.

### Preparation 6. chemical synthesis of macro-molecular polymer-amphotericin B polyconjugate

The method referred to the method used in preparation 3, but instead poly-glucosamine (molecular weight 25,000 Da) was used as the macro-molecular polymer and using 90 mg active drug. 602 mg polyconjugate was obtained. According to the experimental data, in the resultant product, the active drug content =11.2% (w/w), the free active drug content is 0.2%.

### Preparation 7. chemical synthesis of macro-molecule-active drug polyconjugate

The method referred to the method used in preparation 1, but instead nystatin was used as the active drug. According to the experimental data, in the resultant product, the active drug content = 27.1% (w/w), the free active drug content <0.3%.

### Preparation 8. chemical synthesis of macro-molecule-active drug_polyconjugate

The method referred to the method used in preparation 2, but instead natamycin was used as the active drug. According to the experimental data, in the resultant product, the active drug content = 18.7% (w/w), the free active drug content <0.2%.

### Preparation 9. chemical synthesis of macro-molecule-active drug polyconjugate

The method referred to the method used in preparation 3, but instead trichomycin was used as the active drug. According to the experimental data, in the resultant product, the active drug content = 20.4% (w/w), the free active drug content <0.2%.

### Preparation 10. chemical synthesis of macro-molecular polymer-active drug polyconjugate

The method referred to the method used in preparation 1, but instead mepartricin, cannitracin, filipin, aureofungin, etruscomycin, hamycin, perimycin A were used as the active drug, obtaining respectively seven classes of macro-molecular polymer-active drug polyconjugate. In these resultant products, the active drug content are all in the range of 10-35%, while the free active drug content are all <1.0%.

### Preparation 21. chemical synthesis of polyconjugate (001056255, CN1310025A. Ex4)

Polymer of poly (glutamate/aspartic acid, molecular weight 28,000 Da) and paclitaxel: Dissolving the two components in N,N-DMF at ratio 4:1 (molar ratio), using DCC as condensing agent. Dissolving 383 mg poly-dipeptide in 8 ml DMF, adding 8.5 mg N, N-dimethylamino pyridine and 209.4 mg paclitaxel into 152.2 mg DCC, and then adding to the above mixture. Stirring for 22 hours under room temperature. Filtering urea and transferring the resultant solution into chloroform. The product was filtered and redissolved into sodium bicarbonate. After dialyzing against distilled water (cut-off MW 10,000 Da), the product was freeze-dried and weighted 611 mg. According to the experimental data, in the resultant product, the active drug content = 23.8% (w/w), the free active drug content is 3.6%.

### Preparation 22. chemical synthesis of polyconjugate (028121953, CN1596129A, Ex1-Ex2)

Oxidation of hydroxyethyl starch (HES) 130 kD: Placing 10g hydroxyethyl starch (130kD) in a reaction vessel, and dissolving in the minimum amount of water. Adding 2 ml 0. IN iodine solution and about 3 ml 0.1 N NaOH into the solution, stirring (by a magnetic stirring apparatus) until all I₂ reacted and the mixture turned into colorless. Repeating the addition of the iodine solution and/or the NaOH solution for several times, until a total of 10 ml 0.1 N iodine solution and 20 ml 0.1 N NaOH had been added. Then, loading the resultant solution onto a hydrogen ion exchange column (Amberlite IR120), and dialyzing against distilled water for 20 hours in a dialysis tube with exclusion limit of 4-6 kD. Freeze drying and measuring the oxidative degree of the dialyzed product by SOMOGYI method.

Measure of oxidation: Using SOMOGYI method (Meth. Carbohydrate Chem, I,384-386, 1962) to evaluate the obtained oxidized HES (ox-HES). The method is based on measuring the amount of the free aldehydes produced during the reducing process from Cu²⁺to Cu⁺. Iodines and iodates generate iodine which oxidize Cu⁺to Cu²⁺ again, and the excessive iodine will be titrated by hyposulfite.

Synthesis of amphotericin B-hydroxyethyl starch polyconjugate: Dissolving 650 mg (1.5 x 10⁻⁵ mole) dry ox-HES (130 kD, about 100% oxidized) and 2.8 mg (3.0 x 10⁻⁶ mole) amphotericin B in about 4 ml anhydrous DMSO in the reaction vessel, under room temperature and in a nitrogen atmosphere. Stirring the mixture and reacting at 70°C in dark for 24 hours. After adding water with 10 times of volume, the reaction product was dialyzed against water for 48 hours at 4°C in dark, refreshing water 4 times. Freeze drying the product and obtaining light yellow powder. According to the experimental data, in the resultant product, the active drug content = 0.58% (w/w), the free active drug content is 4.8%.

### C, Experimental examples

### Experimental example 1: Solubility assay of the polyconjugate

The solubility of the polyconjugates of the macro-molecular polymer and active drug resulted from the preparations was measured under room temperature (22~25 4°C), using water as solvent. The solubility is characterized by the amount of the soluble active drug therein. For example, when 10 g polyconjugate dissolved in 100 ml water and contained 25% active drug therein, the solubility of the active drug is 2.5%.

According to the experimental data, the polyconjugates of preparation 1 to preparation 9 all have solubility greater than 1.4%, with a range of 1.4-2.8%. For example, the solubility of the polyconjugate of preparation 1 is about 1.95%. However, the solubility of the seven polyconjugates resulted from preparation 10 are all lower than 0.5%, with a range of 0.13~0.47%. For example, the solubility of the poly-aspartic acid-mepartricin polyconjugate is 0.27%.

In addition, the active drug solubility of the product of preparation 21 is 0.53%; the solubility of the active drug amphotericin B of the product resulted from preparation 22 is 0.026%.

### Experimental example 2: Hemolysis assay of the polyconjugate

The hemolysis assay was carried out by reference of the method described in example 3 of CN 1596129 A (FRESENIUS KABI), which was on pages 12-14 of the description. The hemolysis of the test subject was characterized by the concentration of the polyene macrolide substance at the initiation of hemolysis. In the method of the reference, the commercial preparation began to cause hemolysis at a concentration of 0.1 mg/ml amphotericin B, while the amphotericin B-HES polyconjugate began to cause hemolysis at a concentration of 0.4 mg/ml amphotericin B.

According to the experimental data, 11 polyconjugates (polyconjugates of preparation 1 to preparation 9, the mepartricin polyconjugate and cannitracin polyconjugate of preparation 10) had a hemolytic concentration higher than 2.2 mg/ml, all within the range of 2.2-4.6 mg/ml.. For example, the hemolytic concentration of the polyconjugate of preparation 2 was 3.56 mg/ml, indicating a good safety margin of the polyconjugate of the present invention. However, the hemolytic concentrations of the other polyconjugates of preparation 10, and the polyconjugates resulted from preparations 21 and 22 were all lower than 0.7 mg/ml. For example, the hemolytic concentrations of the filipin polyconjugate of preparation 10, the polyconjugate of preparations 21 and the polyconjugate of preparations 22 are 0.45 mg/ml, 0.6 mg/ml, and 0.4 mg/ml, respectively.

### Experimental example 3: In vivo behavior assay of the polyconjugate

The assay was carried out by reference of the method described in ZHAO, Rongsheng, et al. (The pharmacokinetic characteristics of amphotericin B liposomes injection compared with market injection in rabbits after intravenous administration. Journal of Peking University (Health Science), 2001, 33(3):243).

Test samples: three amphotericin B polyconjugates resulted from preparations 1, 2, 3; the control was the amphotericin B liposomes injection (trade name: Amphotec, register No. of the product: H20090963, 100 mg amphotericin B/vial, Three Rivers Co.); they were prepared into sterile solution immediately before use with 5% glucose injection (the polyconjugate of the present invention could be injected after dissolution in 0.9% NaCl injection or 5% glucose injection; the polyconjugate of the present invention had good compatibility with the two solvents; however, the commercial amphotericin B liposomes injection, such as Amphotee, could not be mixed or with 0.9% NaCl injection).

Rabbits, half male and half female (20 total), body weight 2.7-3.2 kg, were randomly divided into groups of 5 per group after overnight fast, and received an intravenous injection of the above four test samples at a dose of 1 mg/kg body weight through the marginal ear veins after fasted overnight. Taking 2 ml blood sample through the marginal ear veins immediately after dosing, at time 0 (30 min before dosing), and at 0.25, 0.5,1, 2, 3, 4, 6, 8, 10, 12, 15, 18, 21, 24, 28, 32, 36 hours after dosing, placing the blood samples into heparin anticoagulant tubes, separating plasma by centrifuging at 3,000 rpm, 10 min. The remaining procedures were identical to those in the reference.

Results: the AUCs of the polyconjugates of Preparations 1,2,3 were 1.62, 1.84, 1.77 times of that of the control, respectively. In addition, time vs. serum concentration curves for each animal were generated, and the duration during which the serum concentration of drug is above 0.5 mg/L was obtained (in ZHAO, Rongsheng, et al., the serum concentration of the commercial amphotericin B injection reduced to 0.5 mg/L 3 hours after dosing, therefore the minimum effective serum concentration is assumed to be 0.5 mg/L herein). For the three polyconjugates resulted from Preparations 1, 2, 3, the durations were 18.4 hr (from 0.82 hr after dosing until 19.2 hr after dosing), 21.8 hr, and 17.41 hr, respectively, while the duration for the control was 11.7 hr (from 1 hr after dosing until 11.7 hr after dosing). Based on the assumption that 0.5 mg/L, is the minimum effective serum concentration of the drug, the polyconjugate of the present invention can maintain a significantly longer period of effective treatment time at the same dosage. Further, it has been surprisingly found that the polyconjugate of the present invention exhibited a blood concentration pattern of the free drug similar to the serum concentration curve obtained by oral administration (e.g., the polyconjugate of Preparation 1 has the serum concentration peak at 7.2 hr), rather than those of the classical injection administrations (e.g., the control preparation has the serum concentration peak at 0 hr), which can avoid the extremely high serum concentration in the early stage of the classical injection administration, thus eliminating or reducing the toxicity or side effect caused by the high concentration of amphotericin B which already has high toxicity by itself

### Experimental example 4: Compatibility Assay

Test subjects: the resultant polyconjugate from Preparation 1 to Preparation 10 of the present invention, the commercial amphotericin B liposomes injection (trade name: Amphotec).

Assay 1: A proper amount of test subject (corresponding to 100 mg active ingredient) was dissolved in 20 ml, 50 ml or 100 ml water for injection, observing the dissolution of the drug; in particular whether anyinsoluble particles were present. Then, the dissolved drug solution was placed in refrigerator at 4°C for 6 hours, observing the dissolution of the drug; in particular whether any insoluble particles were present. Then, the dissolved drug solution was placed at 30°C for 6 hours, observing the dissolution of the drug; in particular whether any insoluble particles were present. The results showed that according to the observation under the three conditions, all the polyconjugates resulted from Preparation 1 to Preparation 10 did not generate any insoluble particle, but Amphotec generated insoluble particles after 6 hours at 30°C.

Assay 2: A proper amount of test subject (corresponding to 100 mg active ingredient) was dissolved in 20 ml, 50 ml or 100 ml 0.9% sodium chloride injection, and the dissolution thereof were observed according to the method of "Assay 1". The results showed that according to the observation under the three conditions, all the polyconjugates resulted from Preparation 1 to Preparation 10 did not generate any insoluble particle, but Amphotec generated insoluble particles after 6 hours at 30°C.

Assay 3: A proper amount of test subject (corresponding to 100 mg active ingredient) was dissolved in 20 ml, 50 ml or 100 ml 5% glucose injection, and the dissolution thereof were observed according to the method of "Assay 1". The results showed that according to the observation under the three conditions, all the polyconjugates resulted from Preparation 1 to Preparation 10 and Amphotec did not generate any insoluble particle.

### Experimental example 5: Drug Safety Assay

It was documented that the LD50 value for intravenous amphotericin B in rat was 1.6 mg/kg (www.pfizer.com/.../Amphotericin%20B%20INJECTION.pdf, MATERIAL SAFETY DATA SHEET, Revision date: 20-Dec-2007,Version: 1.1, Page 3). It was further documented by references that the LD50 of amphotericin B was 2.3 mg/kg in mouse and 1.6 mg/kg in rat (R T Proffitt, A Satorius, S M Chiang, L Sullivan, J P Adler-Moore, JAntimicrobChemother. 1991 Oct ;28 Suppl B: 49-61 1778892 Cit:108).

This experimental example tested the safety of the poly-aspartic acid-amphotericin B polyconjugate resulted from Preparation 1 (can referred to as PA-AB, the active drug content thereof is 23.4%) in animal.

Preparation of the test drug: Dissolving PA-AB in sterile saline to prepare a 5 mg/ml solution (1 ml solution contains 5 mg of equivalent amphotericin B) ready for use.

Female rats were randomly divided into six groups, 3 per group. The control was administered sterile saline (the injection volume is the same as that of 15 mg/kg dosing group), the other five groups were administered 5 mg/kg, 10 mg/kg, 15 mg/kg, 18 mg/kg, 30 mg/kg by intravenous injection, respectively. Animals were weighed at Day 0 (immediately before dosing), and 1, 2, 3, 4, 5, 6, 7, 8 days after dosing. The average body weight for each group and the weight increment expressed as the percentage relative to Day 0 were calculated and showed in the following table:

| Group | Dose of PA-AB* | Body weight g (percentage weight increment compared to Day 0) | | | | | | | | |
|---|---|---|---|---|---|---|---|---|---|---|
| | | 0d | 1d | 2d | 3d | 4d | 5d | 6d | 7d | 8d |
| 1 | 0 | 129.0g | 130.4g | 129.9g | 131.3g | 130.9g | 146.0g | 153.2g | 150.9g | 152.5g |
| 2 | 5mg/kg | 155.5g | 144.0g (-7.4%) | 146.0g (-6.1%) | 143.2g (-7.9%) | 140.2g (-9.8%) | 140.5g (-9.7%) | 143.0g (-8.0%) | 149.0g (-4.2%) | 151.5g (-2.6%) |
| 3 | 10mg/kg | 125.3g | 110.1g (-12.1%) | 114.0g (-9.0%) | 109.0g (-13.0%) | 109.4g (-12.7%) | 116.8g (-6.8%) | 124.5g (-0.6%) | 131.5g (+5.0%) | 133.6g (+6.6%) |
| 4 | 15mg/kg | 145.4g | 129.4g (-11.0%) | 134.3g (-7.6%) | 130.5g (-10.3%) | 125.1g (-14.0%) | 129.3g (-11.1%) | 136.6g (-6.1%) | 145.5g (+0.1%) | 148.3g (+2.0%) |
| 5 | 18mg/kg | 161.3g | 145.0g (-10.1%) | 139.5g (-13.5%) | 1 dead^{#} | 1 dead^{#} | - | - | - | - |
| 6 | 30mg/kg | 139.5g | 125.9g (-9.8%) | 2 dead^{#} | 1 dead^{#} | - | - | - | - | - |

| | | | | | | | | | | |
|---|---|---|---|---|---|---|---|---|---|---|
| Notes: *administered as PA-AB at doses were expressed as amphotericin B equivalent. ^{#}In 18mg/kg dose group, one animal death was observed on Day 3 and Day 4; in 30mg/kg dose group, two animal deaths were observed at Day 2 and another one dead on Day 3; animals in other groups were all alive and regain the initial body weight after completing the experiment. | | | | | | | | | | |

In addition, various batches of the polyconjugates resulted from Preparation 2 to Preparation 10 of the present invention were tested according to the above method, at a dose of 15 mg/kg. Results showed that all animals were alive at Day 8, and regained the initial body weight after completing the experiment. Results of the experiment showed that the polyconjugate of the present invention was not lethal to animal at a dose up to 15 mg/kg; however, the LD50 of conventional amphotericin B was reported to be 1.6 mg/kg, indicating the polyconjugate of the present invention had a much better safety margin.

### Experimental example 6: pharmacodynamic assay

Method: Mice whose immunity was depleted by conventional method (white cell count≤100) were infected with Candida albicans, and randomly divided into eight groups, 20 per group. Mice received the treatment outlined in the following table by injection through the tail vein. The following table showed the groups and doses (in terms of the amount of amphotericin B). Continuously monitoring animal survival for seven days, and counting the number of dead mice on day 7. The results are in the following table:

| Group | Tested drug | Dose | Number of animal death |
|---|---|---|---|
| A | Saline | 0 | 20 dead |
| B1 | amphotericin B | 2 mg/kg | 3 dead |
| B2 | amphotericin B | 1mg/kg | 10 dead |
| C1 | Polyconjugate of preparation 1 | 2 mg/kg | 0 dead |
| C2 | Polyconjugate of preparation 1 | 1 mg/kg | 0 dead |
| C3 | Polyconjugate of preparation 1 | 0.5 mg/kg | 0 dead |
| C4 | Polyconjugate of preparation 1 | 0.25 mg/kg | 5 dead |
| C5 | Polyconjugate of preparation 1 | 0.125 mg/kg | 14 dead |

Results showed that the 50% effective doses of the control groups (animals administered of conventional drug of amphotericin B) were about 1 mg/kg; while the 50% effective dose of the test groups (animals administeredreceived the polyconjugates) were in the ranger of 0.25 mg/kg to 0.125 mg/kg, indicating that the amphotericin B in the polyconjugates of the present invention had therapeutic effect significantly higher than that of the conventional drug.

## Claims

1. Polyconjugate comprising an active drug substance and a macro-molecular polymer, wherein the active drug substance is conjugated with the macro-molecular polymer by chemical bonds.

2. Polyconjugate according to claim 1, wherein the active drug substance is a polyene macrolide antibiotic.

3. Polyconjugate according to claim 1 or 2, wherein the active drug substance is selected from the following polyene macrolide antibiotics: amphotericin B, nystatin, natamycin, trichomycin, mepartricin, cannitracin, filipin, aureofungin, etruscomycin, hamycin, perimycin.

4. Polyconjugate according to any one of claims 1 to 3, wherein the macro-molecular polymer is selected from poly-amino acid formed by polymerizing amino acids, and poly-aminohexose formed by polymerizing aminohexoses.

5. Polyconjugate according to any one of claims 1 to 4, wherein the macro-molecular polymer is poly-amino acid formed by polymerizing amino acids, the amino acid is selected from carboxylic acid-type amino acid, amino-type amino acid, or the combination thereof

6. Polyconjugate according to claim 5, wherein the carboxylic acid-type amino acid is selected from aspartic acid (Asp), glutamate (Glu), or the combinations thereof at any proportion.

7. Polyconjugate according to claim 5, wherein the amino-type amino acid is selected from ornithine (Orn), lysine (Lys), arginine (Arg), hydroxylysine (Hyl), or the combinations thereof at any proportion.

8. Polyconjugate according to any one of claims 4 to 7, wherein the amino acids in the poly-amino acid comprise D-amino acid, L-amino acid, and DL-amino acid.

9. Polyconjugate according to claim 4, wherein the aminohexose is selected from glucosamine, galactosamine, acetyl glucosamine, acetyl galactosamine, or the combinations thereof

10. Polyconjugate according to any one of claims 4 to 9, wherein the average molecular weight of the poly-amino acid is 5000-100000 daltons.

11. Polyconjugate according to any one of claims 4 to 9, wherein the average molecular weight of the poly-aminohexose is 10000-200000 daltons.

12. Polyconjugate according to any one of claims 1 to 11, wherein the weight of the active drug substance accounts for 5-60% of the total weight of the polyconjugate.

13. Method of preparing the polyconjugates according to any one of claims 1 to 12, including the following steps:
i) providing the macro-molecular polymer, dissolving into a solvent to form solution;
ii) adding and dissolving the active drug substance into the solution obtained in step (i);
iii) adding catalyst and condensing agent into the solution obtained in step (ii), condensing the macro-molecular polymer with the active drug substance to form the polyconjugate thereof, wherein the macro-molecular polymer and the active drug substance are combining with each other by chemical bonds;
(iv) separating and extracting the obtained polyconjugate.

14. Method according to claim 13, wherein the solvent in step (i) is an organic solvent, such as dimethylformamide, dimethyl sulfoxide.

15. Method according to claim 13, wherein the catalyst in step (iii) is 4-dimethylaminopyridine.

16. Method according to claim 13, wherein the condensing agent in step (iii) is dicyclohexylcarbodiimide.

17. Method of increasing the solubility of the active drug substance, comprising the step of combining the active drug substance with the macro-molecular polymer by chemical bonds to form the polyconjugate.

18. Method according to claim 17, wherein the active drug substance is a polyene macrolide antibiotic.

19. Method according to claim 17, wherein the active drug substance is selected from the following polyene macrolide antibiotics: amphotericin B, nystatin, natamycin, trichomycin, mepartricin, cannitracin, filipin, aureofungin, etruscomycin, hamycin, perimycin.

20. Method according to claim 17, wherein the macro-molecular polymer is selected from poly-amino acid formed by polymerizing amino acids, and poly-aminohexose formed by polymerizing aminohexoses.

21. Method according to claim 20, wherein the macro-molecular polymer is poly-amino acid formed by polymerizing amino acids, the amino acid is selected from carboxylic acid-type amino acid, amino-type amino acid, or the combination thereof..

22. Method according to claim 21, wherein the carboxylic acid-type amino acid is selected from aspartic acid (Asp), glutamate (Glu), or the combinations thereof at any proportion.

23. Method according to claim 21, wherein the the amino-type amino acid is selected from ornithine (Orn), lysine (Lys), arginine (Arg), hydroxylysine (Hyl), or the combinations thereof at any proportion.

24. Method according to claim 20, wherein the amino acids in the poly-amino acid comprise D-amino acid, L-amino acid, and DL-amino acid.

25. Method according to claim 20, wherein the aminohexose is selected from glucosamine, galactosamine, acetyl glucosamine, acetyl galactosamine, or the combinations thereof

26. Method according to any one of claims 17 to 25, wherein the average molecular weight of the poly-amino acid is 5000-100000 daltons.

27. Method according to any one of claims 17 to 25, wherein the average molecular weight of the poly-aminohexose is 10000-200000 daltons.

28. Method according to any one of claims 17 to 27, wherein the weight of the active drug substance accounts for 5-60% of the total weight of the polyconjugate.

29. Method according to any one of claims 17 to 28, wherein the step of combining the active drug substance with the macro-molecular polymer by chemical bonds to form the polyconjugate is carried out in accordance with the method according to any one of claims 13 to 16.

30. Pharmaceutical composition, comprising the polyconjugate according to any one of claims 1 to 12, and optionally any pharmaceutically acceptable auxiliary materials.
